# EUROPEAN PATENT APPLICATION

(11) **EP 4 424 313 A1**
(43) Date of publication of application: **04.09.2024**
(21) Application number: 22887110.9
(22) Date of filing: 27.10.2022
(51) Int. Cl.: A61K 31/5377, A61K 9/10, A61K 47/02, A61K 47/12, A61K 47/18, A61K 47/20, A61K 47/22, A61K 47/26, A61K 47/36, A61K 47/38, A61K 47/46, A61P 35/00, A61P 43/00

(54) **SYRUP PREPARATION**

(30) Priority: 28.10.2021 JP 2021176595
(71) Applicant: CHUGAI SEIYAKU KABUSHIKI KAISHA, Tokyo 115-8543 (JP)
(72) Inventor: KANOH Takeo, Tokyo 115-8543 (JP); TAKAHASHI Shodai, Tokyo 115-8543 (JP); MAEDA Yuki, Tokyo 115-8543 (JP); HASHIMOTO Shota, Tokyo 115-8543 (JP)
(74) Representative: Vossius & Partner Patentanwälte Rechtsanwälte mbB
(86) International application number: PCT/JP2022/040113
(87) International publication number: WO 2023/074785

(57) **Abstract**

The present invention relates to a syrup containing alectinib or a salt thereof. The present invention provides a syrup containing alectinib or a salt thereof, which is a poorly water-soluble agent, and having improved fluidity and/or palatability. The syrup of the present invention has a plasma concentration profile equivalent to or greater than, or is biologically equivalent to, a capsule containing the same amount of alectinib or a salt thereof as the syrup, or has an enhanced oral bioavailability compared to a capsule containing the same amount of alectinib or a salt thereof as the syrup.

## Description

### [Technical Field]

The present invention relates to a syrup containing a poorly water-soluble agent, in particular, alectinib or a salt thereof.

### [Background Art]

Anaplastic Lymphoma Kinase (ALK) is one of the receptor-type tyrosine kinases belonging to the insulin receptor family (Non Patent Literatures 1 and 2). It has been reported that the genetic abnormality of ALK causes the production of abnormal kinases fused with other genes.

As diseases with abnormalities of ALK, cancer and cancer metastasis (Non-Patent Literature 1, Patent Literature 1), depression, cognitive dysfunction (Non-Patent Literature 2) and the like are known. For those diseases, inhibitors of ALK provides effective therapeutic and prophylactic drugs.

Alectinib is known as a tetracyclic compound having a morpholine backbone represented by the following formula (I) (compound name: 9-ethyl-6,6-dimethyl-8-(4-morpholine-4-yl-piperidine-1-yl)-11-oxo-6,11-dihydro-5H-benzo[b]carbazole-3-carbonitrile) 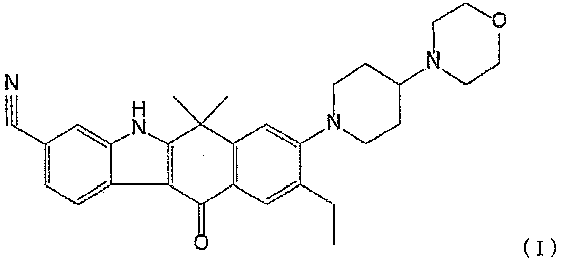 (Patent Literatures 2, 3, 4, and 5). Alectinib hydrochloride is used worldwide as a second-generation Anaplastic Lymphoma Kinase (ALK) inhibitor and as a therapeutic agent for cancers with ALK abnormalities.

ALK abnormalities have been widely observed in various pediatric malignant solid tumors. In pediatric cancers, ALK inhibitors are also considered to be an important target in the development of therapeutics for cancers with ALK abnormalities. A study for evaluating the efficacy of alectinib has been conducted for rare cancers including pediatric cancers with ALK abnormalities (Non Patent Literature). However, since the preparation provided in the study is a capsule, it is difficult for children to swallow the preparation. For example, children under 2 years of age received it by way of a solution obtained by suspending the whole capsule in water which administered via a tube (Non Patent Literature 3).

Alectinib is a poorly water-soluble basic drug. In producing the preparation containing alectinib, the followings are reported: a dissolution aid is allowed to be present together with alectinib to prepare a composition (Patent Literature 3); granules containing alectinib or a salt thereof are formed, and allowed to be present together with a disintegrant to prepare a capsule with good dissolution property (Patent Literature 5); and a basic substance is allowed to be present together with alectinib or a salt thereof and a surfactant to produce a preparation having good disintegration and dissolution properties (Patent Literature 6).

Patent Literature 1 :JP2009-100783 A
Patent Literature 2 :Japanese Patent No. 4588121
Patent Literature 3 :Japanese Patent No. 4918630
Patent Literature 4 :Japanese Patent No. 5006987
Patent Literature 5 :Japanese Patent No. 5859712
Patent Literature 6 :WO2020/004630

Non Patent Literature 1 :Nature, Volume 448, Pages 561-566, 2007
Non Patent Literature 2 :Neuropsychopharmacology, Volume 33, Pages 685-700, 2008
Non Patent Literature 3 :JMACCT ID JMA-IIA00364, Physician-Led Clinical Trial of Alectinib in Rare Cancers with ALK Gene Abnormalities (https://dbcentre3.jmacct.med.or.jp/JMACTR/App/JMACTRE02_04/JMACTRE02_ 04.aspx?kbn=3&seqno=8259)

### [Summary of Invention]

In the situations described above, it is desired to provide a syrup with good fluidity and/or palatability regarding poorly water-soluble agents such as alectinib hydrochloride. Further for the syrup containing alectinib or a salt thereof, it is desired to provide a syrup which has a desired plasma concentration profile, or is biologically equivalent to, a capsule containing the same amount of alectinib or a salt thereof as the syrup, or has an enhanced oral bioavailability compared to a capsule containing the same amount of alectinib or a salt thereof as the syrup.

The present inventors have found that a syrup having good fluidity and/or palatability can be obtained by further allowing an inhibitor for gel-like substances to be present together in a composition containing a poorly water-soluble agent such as alectinib hydrochloride and a surfactant, or to be present together with the poorly water-soluble agent and/or the surfactant. The syrup of the present invention includes a syrup of the present invention which has a plasma concentration profile equivalent to or greater than, or is biologically equivalent to, a capsule containing the same amount of alectinib or a salt thereof as the syrup, or having enhanced oral bioavailability compared to a capsule containing the same amount of alectinib or a salt thereof as the syrup.

That is, the present invention includes the following aspects.

One aspect of the present invention is as follows.
(A-1) A syrup containing alectinib or a salt thereof, wherein the syrup has a plasma concentration profile equivalent to or greater than a capsule containing the same amount of alectinib or a salt thereof as the syrup.
(A-2) A syrup containing alectinib or a salt thereof, wherein the syrup has AUC and Cₘₐₓ equivalent to or greater than a capsule containing the same amount of alectinib or a salt thereof as the syrup.
(A-3) A syrup containing alectinib or a salt thereof, wherein the syrup has bioequivalence to a capsule containing the same amount of alectinib or a salt thereof as the syrup.
(A-4) The syrup according to any one of (A-1) to (A-3), further containing an inhibitor for gel-like substances.
(A-5) The syrup according to (A-4), wherein a weight ratio of the alectinib or a salt thereof and the inhibitor for gel-like substances is from 100 : 30 to 100 : 90 in terms of free form.
(A-6) The syrup according to any one of (A-1) to (A-5), further containing a surfactant and an inhibitor for gel-like substances.
(A-7) The syrup according to (A-6), wherein the inhibitor for gel-like substances is a salt.
(A-8) The syrup according to (A-7), wherein the salt is a water-soluble salt.
(A-9) The syrup according to (A-7) or (A-8), wherein the salt is an inorganic salt or an organic salt.
(A-10) The syrup according to (A-9), wherein the inorganic salt is selected from the group consisting of a chloride salt, a sulfate, a hydroxide salt, a carbonate, and a bicarbonate.
(A-11) The syrup according to (A-9), wherein the organic salt is selected from the group consisting of a tartrate, an acetate, a citrate, an amino acid salt, an alginate, and a sorbate.
(A-12) The syrup according to any one of (A-7) to (A-10), wherein the salt is selected from the group consisting of calcium chloride, sodium chloride, potassium chloride, sodium sulfate, calcium sulfate, calcium dihydrogen phosphate, sodium hydroxide, calcium hydroxide, and sodium bicarbonate.
(A-13) The syrup according to any one of (A-6) to (A-12), wherein the surfactant is an anionic surfactant.
(A-14) The syrup according to (A-13), wherein the anionic surfactant is sodium lauryl sulfate.
(A-15) The syrup according to any one of (A-6) to (A-14), further containing a pH adjusting agent.
(A-16) The syrup according to (A-15), wherein the pH adjusting agent is tartaric acid.
(A-17) The syrup according to any one of (A-6) to (A-16), further containing one or more substances selected from a disintegrant, an excipient, and a binder.
(A-18) The syrup according to any one of (A-6) to (A-17), wherein a weight ratio of the alectinib or a salt thereof and the inhibitor for gel-like substances is from 100 : 30 to 100 : 90 in terms of free form.
(A-19) The syrup according to any one of (A-6) to (A-18), wherein a weight ratio of the alectinib or a salt thereof and the surfactant and the inhibitor for gel-like substances is from 100 : 3 : 30 to 100 : 50 : 90 in terms of free form.
(A-20) The syrup according to any one of (A-6) to (A-19), further containing one or more substances selected from a preservative, a thickener, a sweetener, and a flavor.
(A-21) The syrup according to (A-20), wherein the thickener is selected from the group consisting of xanthan gum, hydroxyethyl cellulose, and sodium carboxymethyl cellulose; the sweetener is selected from the group consisting of sucrose, mannitol, fructose, lactose, sodium saccharin, aspartame, xylitol, erythritol, sucralose, and acesulfame potassium; and the flavor is selected from the group consisting of a strawberry flavor, a raspberry flavor, an orange flavor, an apple flavor, a cherry black flavor, and a yogurt flavor.
(A-22) The syrup according to any one of (A-1) to (A-21), wherein the syrup is a dry syrup.

Another aspect of the present invention is as follows.
(B-1) A syrup containing alectinib or a salt thereof, a surfactant, and an inhibitor for gel-like substances.
(B-2) The syrup according to (B-1), wherein the inhibitor for gel-like substances is a salt.
(B-3) The syrup according to (B-2), wherein the salt is a water-soluble salt.
(B-4) The syrup according to (B-2) or (B-3), wherein the salt is an inorganic salt or an organic salt.
(B-5) The syrup according to (B-4), wherein the inorganic salt is selected from the group consisting of a chloride salt, a sulfate, a hydroxide salt, a carbonate, and a bicarbonate.
(B-6) The syrup according to (B-4), wherein the organic salt is selected from the group consisting of a tartrate, an acetate, a citrate, an amino acid salt, an alginate, and a sorbate.
(B-7) The syrup according to any one of (B-2) to (B-5), wherein the salt is selected from the group consisting of calcium chloride, sodium chloride, potassium chloride, sodium sulfate, calcium sulfate, calcium dihydrogen phosphate, sodium hydroxide, calcium hydroxide, and sodium bicarbonate.
(B-8) The syrup according to any one of (B-1) to (B-7), wherein the surfactant is an anionic surfactant.
(B-9) The syrup according to (B-8), wherein the anionic surfactant is sodium lauryl sulfate.
(B-10) The syrup according to any one of (B-1) to (B-9), further containing a pH adjusting agent.
(B-11) The syrup according to (B-10), wherein the pH adjusting agent is tartaric acid.
(B-12) The syrup according to any one of (B-1) to (B-11), further containing one or more substances selected from a disintegrant, an excipient, and a binder.
(B-13) The syrup according to any one of (B-1) to (B-12), wherein a weight ratio of the alectinib or a salt thereof and the inhibitor for gel-like substances is from 100 : 30 to 100 : 90 in terms of free form.
(B-14) The syrup according to any one of (B-1) to (B-13), wherein a weight ratio of the alectinib or a salt thereof and the surfactant and the inhibitor for gel-like substances is from 100 : 3 : 30 to 100 : 50 : 90 in terms of free form.
(B-15) The syrup according to any one of (B-1) to (B-14), further containing one or more substances selected from a preservative, a thickener, a sweetener, and a flavor.
(B-16) The syrup according to (B-15), wherein the thickener is selected from the group consisting of xanthan gum, hydroxyethyl cellulose, and sodium carboxymethyl cellulose; the sweetener is selected from the group consisting of sucrose, mannitol, fructose, lactose, sodium saccharin, aspartame, xylitol, erythritol, sucralose, and acesulfame potassium; and the flavor is selected from the group consisting of a strawberry flavor, a raspberry flavor, an orange flavor, an apple flavor, a cherry black flavor, and a yogurt flavor.
(B-17) The syrup according to any one of (B-1) to (B-16), wherein the syrup is a dry syrup.

Yet another aspect of the present invention is as follows.
(C-1) A syrup containing alectinib or a salt thereof and an inhibitor for gel-like substances, the syrup having an enhanced oral bioavailability.
(C-2) The syrup according to (C-1), wherein the syrup has the enhanced oral bioavailability compared to a capsule containing the same amount of alectinib or a salt thereof as the syrup.
(C-3) The syrup according to (C-1), further containing a surfactant.
(C-4) The syrup according to any one of (C-1) to (C-5), further containing a surfactant and an inhibitor for gel-like substances.
(C-5) The syrup according to (C-4), wherein the inhibitor for gel-like substances is a salt.
(C-6) The syrup according to (C-5), wherein the salt is a water-soluble salt.
(C-7) The syrup according to (C-5) or (C-6), wherein the salt is an inorganic salt or an organic salt.
(C-8) The syrup according to (C-7), wherein the inorganic salt is selected from the group consisting of a chloride salt, a sulfate, a hydroxide salt, a carbonate, and a bicarbonate.
(C-9) The syrup according to (C-7), wherein the organic salt is selected from the group consisting of a tartrate, an acetate, a citrate, an amino acid salt, an alginate, and a sorbate.
(C-10) The syrup according to any one of (C-5) to (C-8), wherein the salt is selected from the group consisting of calcium chloride, sodium chloride, potassium chloride, sodium sulfate, calcium sulfate, calcium dihydrogen phosphate, sodium hydroxide, calcium hydroxide, and sodium bicarbonate.
(C-11) The syrup according to any one of (C-4) to (C-10), wherein the surfactant is an anionic surfactant.
(C-12) The syrup according to (C-11), wherein the anionic surfactant is sodium lauryl sulfate.
(C-13) The syrup according to any one of (C-4) to (C-12), further containing a pH adjusting agent.
(C-14) The syrup according to (C-13), wherein the pH adjusting agent is tartaric acid.
(C-15) The syrup according to any one of (C-4) to (C-14), further containing one or more substances selected from a disintegrant, an excipient, and a binder.
(C-16) The syrup according to any one of (C-4) to (C-15), wherein a weight ratio of the alectinib or a salt thereof and the inhibitor for gel-like substances is from 100 : 30 to 100 : 90 in terms of free form.
(C-17) The syrup according to any one of (C-4) to (C-16), wherein a weight ratio of the alectinib or a salt thereof and the surfactant and the inhibitor for gel-like substances is from 100 : 3 : 30 to 100 : 50 : 90 in terms of free form.
(C-18) The syrup according to any one of (C-4) to (C-17), further containing one or more substances selected from a preservative, a thickener, a sweetener, and a flavor.
(C-19) The syrup according to (C-18), wherein the thickener is selected from the group consisting of xanthan gum, hydroxyethyl cellulose, and sodium carboxymethyl cellulose; the sweetener is selected from the group consisting of sucrose, mannitol, fructose, lactose, sodium saccharin, aspartame, xylitol, erythritol, sucralose, and acesulfame potassium; and the flavor is selected from the group consisting of a strawberry flavor, a raspberry flavor, an orange flavor, an apple flavor, a cherry black flavor, and a yogurt flavor.

The present invention also includes the following aspects.
(1) A syrup containing alectinib or a salt thereof, a surfactant, and an inhibitor for gel-like substances.
(2) The syrup according to (1), wherein the inhibitor for gel-like substances is a salt.
(3) The syrup according to (2), wherein the salt is a water-soluble salt.
(4) The syrup according to (2) or (3), wherein the salt is an inorganic salt or an organic salt.
(4a1) The syrup according to (2) or (3), wherein the salt is an inorganic salt or organic salt of a metal.
(5) The syrup according to (4), wherein the inorganic salt is selected from the group consisting of a chloride salt, a sulfate, a hydroxide salt, a carbonate, and a bicarbonate.
(5a) The syrup according to (4a1), wherein the inorganic salt of a metal is a halide salt, a carbonate, a bicarbonate, a sulfate or a hydroxide salt of a metal.
(5a1) The syrup according to (2) or (3), wherein the salt is selected from the group consisting of calcium chloride, sodium chloride, potassium chloride, magnesium chloride, sodium sulfate, calcium sulfate, calcium dihydrogen phosphate, dipotassium phosphate, sodium hydroxide, calcium hydroxide, sodium bicarbonate, potassium bicarbonate, ammonium carbonate, sodium tartrate, sodium acetate, sodium citrate, sodium glutamate, magnesium aspartate, sodium alginate, and potassium sorbate.
(6) The syrup according to (4), wherein the organic salt is selected from the group consisting of a tartrate, an acetate, a citrate, an amino acid salt, an alginate, and a sorbate.
(7) The syrup according to any one of (2) to (5), wherein the salt is selected from the group consisting of calcium chloride, sodium chloride, potassium chloride, sodium sulfate, calcium sulfate, calcium dihydrogen phosphate, sodium hydroxide, calcium hydroxide, and sodium bicarbonate.
(8) The syrup according to any one of (1) to (7), wherein the surfactant is an anionic surfactant.
(9) The syrup according to (8), wherein the anionic surfactant is sodium lauryl sulfate.
(10) The syrup according to any one of (1) to (9), further containing a pH adjusting agent.
(10a1) The syrup according to (10), wherein the pH adjusting agent is selected from the group consisting of tartaric acid, citric acid, and maleic acid.
(11) The syrup according to (10), wherein the pH adjusting agent is tartaric acid.
(12) The syrup according to any one of (1) to (11), further containing one or more substances selected from a disintegrant, an excipient, and a binder.
(12a1) The syrup according to (12), wherein the disintegrant is selected from the group consisting of carmellose calcium, crospovidone, sodium starch glycolate and croscarmellose sodium; the excipient is selected from the group consisting of starch, lactose hydrate and crystalline cellulose; and the binder is selected from the group consisting of hydroxypropyl cellulose, hydroxypropyl methyl cellulose, methyl cellulose, povidone (polyvinylpyrrolidone) and gum arabic powder.
(12a2) The syrup according to (12), wherein the syrup contains one or more substances selected from crospovidone as the disintegrant, lactose hydrate as the excipient, and hydroxypropyl cellulose as the binder.
(13) The syrup according to any one of (1) to (8), wherein a weight ratio of the alectinib or a salt thereof and the inhibitor for gel-like substances is from 100 : 10 to 100 : 70 in terms of free form.
(13a1) The syrup according to any one of (1) to (13), wherein a weight ratio of the alectinib or a salt thereof and the surfactant and the inhibitor for gel-like substances is from 100 : 3 : 30 to 100 : 50 : 90 in terms of free form.
(14) The syrup according to any one of (1) to (9), further containing one or more substances selected from a preservative, a thickener, a sweetener, and a flavor.
(15) The syrup according to (14), wherein the thickener is selected from the group consisting of xanthan gum, hydroxyethyl cellulose, and sodium carboxymethyl cellulose; the sweetener is selected from the group consisting of sucrose, mannitol, fructose, lactose, sodium saccharin, aspartame, xylitol, erythritol, sucralose, and acesulfame potassium; and the flavor is selected from the group consisting of a strawberry flavor, a raspberry flavor, an orange flavor, an apple flavor, a cherry black flavor, and a yogurt flavor.
(16) The syrup according to any one of (1) to (15), wherein the syrup is a dry syrup.
(17) A dry syrup containing alectinib or a salt thereof, sodium lauryl sulfate, and calcium chloride.
(18) A dry syrup containing alectinib or a salt thereof, sodium lauryl sulfate, calcium chloride, and tartaric acid.
(19) A dry syrup containing alectinib or a salt thereof, sodium lauryl sulfate, calcium chloride, tartaric acid, and crospovidone.
(20) A dry syrup containing alectinib or a salt thereof, sodium lauryl sulfate, calcium chloride, tartaric acid, crospovidone, sodium benzoate, sucralose, lactose hydrate, and hydroxypropyl cellulose.
(21) A dry syrup containing alectinib or a salt thereof, sodium lauryl sulfate, calcium chloride, tartaric acid, crospovidone, sodium benzoate, sucralose, lactose hydrate, hydroxypropyl cellulose, xanthan gum, and strawberry flavor.

Another aspect of the present invention includes a method for suppressing a decrease in fluidity of a pharmaceutical composition containing alectinib or a salt thereof and a surfactant.
(1b) A method for suppressing a decrease in fluidity of a pharmaceutical composition containing alectinib or a salt thereof and a surfactant, the method comprising allowing an inhibitor for gel-like substances to be present together in the pharmaceutical composition.
(2b) The method according to (1b), wherein the inhibitor for gel-like substances is a salt.
(3b) The method according to (2b), wherein the salt is a water-soluble salt.
(4b) The method according to (2b) or (3b), wherein the salt is an inorganic salt or an organic salt.
(4b1) The method according to (2b) or (3b), wherein the salt is an inorganic salt or organic salt of a metal.
(4b2) The method according to (2b) or (3b), wherein the salt is selected from the group consisting of calcium chloride, sodium chloride, potassium chloride, magnesium chloride, sodium sulfate, calcium sulfate, calcium dihydrogen phosphate, dipotassium phosphate, sodium hydroxide, calcium hydroxide, sodium bicarbonate, potassium bicarbonate, ammonium carbonate, sodium tartrate, sodium acetate, sodium citrate, sodium glutamate, magnesium aspartate, sodium alginate, and potassium sorbate.
(5b) The method according to (4b), wherein the inorganic salt is selected from the group consisting of a chloride salt, a sulfate, a hydroxide salt, a carbonate, and a bicarbonate.
(5b1) The method according to (4b1), wherein the inorganic salt of a metal is selected from the group consisting of a chloride salt, a sulfate, a hydroxide salt, a carbonate, and a bicarbonate.
(6b) The method according to (4b) or (4b'), wherein the organic salt is selected from the group consisting of a tartrate, an acetate, a citrate, an amino acid salt, an alginate, and a sorbate.
(7b) The method according to any one of (2b) to (5b), wherein the salt is selected from the group consisting of calcium chloride, sodium chloride, potassium chloride, sodium sulfate, calcium sulfate, calcium dihydrogen phosphate, sodium hydroxide, calcium hydroxide, and sodium bicarbonate.
(8b) The method according to any one of (1b) to (7b), wherein the surfactant is an anionic surfactant.
(9b) The method according to (8b), wherein the anionic surfactant is sodium lauryl sulfate.
(10b) The method according to any one of (1b) to (9b), wherein the pharmaceutical composition further contains a pH adjusting agent.
(10b1) The method according to (10b), wherein the pH adjusting agent is selected from the group consisting of tartaric acid, citric acid, and maleic acid.
(11b) The method according to (10b), wherein the pH adjusting agent is tartaric acid.
(12b) The method according to any one of (1b) to (11b), wherein the pharmaceutical composition further contains one or more substances selected from a disintegrant, an excipient, and a binder.
(12b1) The method according to (12b), wherein the disintegrant is selected from the group consisting of carmellose calcium, crospovidone, sodium starch glycolate and croscarmellose sodium; the excipient is selected from the group consisting of starch, lactose hydrate and crystalline cellulose; and the binder is selected from the group consisting of hydroxypropyl cellulose, hydroxypropyl methyl cellulose, methyl cellulose, povidone (polyvinylpyrrolidone) and gum arabic powder.
(12b2) The method according to (12b), wherein the pharmaceutical composition contains one or more substances selected from crospovidone as the disintegrant, lactose hydrate as the excipient, and hydroxypropyl cellulose as the binder.
(13b) The method according to any one of (1b) to (8b), wherein a weight ratio of the alectinib or a salt thereof and the inhibitor for gel-like substances is from 100 : 10 to 100 : 70 in terms of free form.
(13b1) The syrup according to any one of (1b) to (13b), wherein a weight ratio of the alectinib or a salt thereof and the surfactant and the inhibitor for gel-like substances is from 100 : 3 : 30 to 100 : 50 : 90 in terms of free form.
(14b) The method according to any one of (1b) to (9b), wherein the pharmaceutical composition further contains one or more substances selected from a preservative, a thickener, a sweetener, and a flavor.
(15b) The method according to (14b), wherein the thickener is selected from the group consisting of xanthan gum, hydroxyethyl cellulose, and sodium carboxymethyl cellulose; the sweetener is selected from the group consisting of sucrose, mannitol, fructose, lactose, sodium saccharin, aspartame, xylitol, erythritol, sucralose, and acesulfame potassium; and the flavor is selected from the group consisting of a strawberry flavor, a raspberry flavor, an orange flavor, an apple flavor, a cherry black flavor, and a yogurt flavor.
(16b1) The method according to any one of (1b) to (15b), wherein the pharmaceutical composition is a syrup.
(16b2) The method according to any one of (1b) to (15b), wherein the pharmaceutical composition is a dry syrup.

Yet another aspect of the present invention includes a method for producing a syrup containing alectinib or a salt thereof.
(1c) A method for producing a syrup containing a pharmaceutical composition containing alectinib or a salt thereof and a surfactant, the method comprising allowing an inhibitor for gel-like substances to be present together in the pharmaceutical composition.
(1c-1) A method for producing a syrup containing a pharmaceutical composition containing alectinib or a salt thereof, the method comprising allowing an inhibitor for gel-like substances to be present together in the pharmaceutical composition, wherein the syrup has a plasma concentration profile equivalent to or greater than a capsule containing the same amount of alectinib or a salt thereof as the syrup.
(1c-2) A method for producing a syrup containing a pharmaceutical composition containing alectinib or a salt thereof, the method comprising allowing an inhibitor for gel-like substances to be present together in the pharmaceutical composition, wherein the syrup has AUC equivalent to or greater than a capsule containing the same amount of alectinib or a salt thereof as the syrup.
(1c-3) A method for producing a syrup containing a pharmaceutical composition containing alectinib or a salt thereof, the method comprising allowing an inhibitor for gel-like substances to be present together in the pharmaceutical composition, wherein the syrup has bioequivalence to a capsule containing the same amount of alectinib or a salt thereof as the syrup.
(1c-4) The method according to any one of (1c-1) to (1c-3), wherein the pharmaceutical composition further contains a surfactant.
(2c) The method according to any one of (1c) to (1c-4), wherein the inhibitor for gel-like substances is a salt.
(3c) The method according to (2c), wherein the salt is a water-soluble salt.
(4c) The method according to (2c) or (3c), wherein the salt is an inorganic salt or an organic salt.
(4c1) The method according to (2c) or (3c), wherein the salt is an inorganic salt or organic salt of a metal.
(4c2) The method according to (2c) or (3c), wherein the salt is selected from the group consisting of calcium chloride, sodium chloride, potassium chloride, magnesium chloride, sodium sulfate, calcium sulfate, calcium dihydrogen phosphate, dipotassium phosphate, sodium hydroxide, calcium hydroxide, sodium bicarbonate, potassium bicarbonate, ammonium carbonate, sodium tartrate, sodium acetate, sodium citrate, sodium glutamate, magnesium aspartate, sodium alginate, and potassium sorbate.
(5c) The method according to (4c), wherein the inorganic salt is selected from the group consisting of a chloride salt, a sulfate, a hydroxide salt, a carbonate, and a bicarbonate.
(5c1) The method according to (4c1), wherein the inorganic salt of a metal is selected from the group consisting of a chloride salt, a sulfate, a hydroxide salt, a carbonate, and a bicarbonate.
(6c) The method according to (4c), wherein the organic salt is selected from the group consisting of a tartrate, an acetate, a citrate, an amino acid salt, an alginate, and a sorbate.
(7c) The method according to any one of (2c) to (5c), wherein the salt is selected from the group consisting of calcium chloride, sodium chloride, potassium chloride, sodium sulfate, calcium sulfate, calcium dihydrogen phosphate, sodium hydroxide, calcium hydroxide, and sodium bicarbonate.
(8c) The method according to any one of (1c) to (7c), wherein the surfactant is an anionic surfactant.
(9c) The method according to (8c), wherein the anionic surfactant is sodium lauryl sulfate.
(10c) The method according to any one of (1c) to (9c), wherein the pharmaceutical composition further contains a pH adjusting agent.
(10c1) The method according to (10c), wherein the pH adjusting agent is selected from the group consisting of tartaric acid, citric acid, and maleic acid.
(11c) The method according to (10c), wherein the pH adjusting agent is tartaric acid.
(12c) The method according to any one of (1c) to (11c), wherein the pharmaceutical composition further contains one or more substances selected from a disintegrant, an excipient, and a binder.
(12c1) The method according to (12c), wherein the disintegrant is selected from the group consisting of carmellose calcium, crospovidone, sodium starch glycolate and croscarmellose sodium; the excipient is selected from the group consisting of starch, lactose hydrate and crystalline cellulose; and the binder is selected from the group consisting of hydroxypropyl cellulose, hydroxypropyl methyl cellulose, methyl cellulose, povidone (polyvinylpyrrolidone) and gum arabic powder.
(12c2) The method according to (12c), wherein the pharmaceutical composition contains one or more substances selected from crospovidone as the disintegrant, lactose hydrate as the excipient, and hydroxypropyl cellulose as the binder.
(13c) The method according to any one of (1e) to (12c2), wherein a weight ratio of the alectinib or a salt thereof and the inhibitor for gel-like substances is from 100 : 10 to 100 : 70 in terms of free form.
(13c1) The syrup according to any one of (1c) to (13c), wherein a weight ratio of the alectinib or a salt thereof and the surfactant and the inhibitor for gel-like substances is from 100 : 3 : 30 to 100 : 50 : 90 in terms of free form.
(14c) The method according to any one of (1c) to (13c1), wherein the pharmaceutical composition further contains one or more substances selected from a preservative, a thickener, a sweetener, and a flavor.
(15c) The method according to (14c), wherein the thickener is selected from the group consisting of xanthan gum, hydroxyethyl cellulose, and sodium carboxymethyl cellulose; the sweetener is selected from the group consisting of sucrose, mannitol, fructose, lactose, sodium saccharin, aspartame, xylitol, erythritol, sucralose, and acesulfame potassium; and the flavor is selected from the group consisting of a strawberry flavor, a raspberry flavor, an orange flavor, an apple flavor, a cherry black flavor, and a yogurt flavor.
(16c) The method according to any one of (1c) to (15c), wherein the pharmaceutical composition is a dry syrup.
(17c) The method according to any one of (1c) to (16c), wherein the pharmaceutical composition does not result in a decrease in fluidity when suspended.

Yet another aspect of the present invention includes a method for producing a syrup containing alectinib or a salt thereof, wherein the syrup has an enhanced oral bioavailability.
(1d) A method for producing a syrup containing a pharmaceutical composition containing alectinib or a salt thereof, the method comprising allowing an inhibitor for gel-like substances to be present together in the pharmaceutical composition, wherein the syrup has an enhanced oral bioavailability.
(2d) The method according to (1d), wherein the inhibitor for gel-like substances is a salt.
(3d) The method according to (2d), wherein the salt is a water-soluble salt.
(4d) The method according to (2d) or (3d), wherein the salt is an inorganic salt or an organic salt.
(4d1) The method according to (2d) or (3d), wherein the salt is an inorganic salt or organic salt of a metal.
(4d2) The method according to (2d) or (3d), wherein the salt is selected from the group consisting of calcium chloride, sodium chloride, potassium chloride, magnesium chloride, sodium sulfate, calcium sulfate, calcium dihydrogen phosphate, dipotassium phosphate, sodium hydroxide, calcium hydroxide, sodium bicarbonate, potassium bicarbonate, ammonium carbonate, sodium tartrate, sodium acetate, sodium citrate, sodium glutamate, magnesium aspartate, sodium alginate, and potassium sorbate.
(5d) The method according to (4d), wherein the inorganic salt is selected from the group consisting of a chloride salt, a sulfate, a hydroxide salt, a carbonate, and a bicarbonate.
(5d1) The method according to (4d1), wherein the inorganic salt of a metal is selected from the group consisting of a chloride salt, a sulfate, a hydroxide salt, a carbonate, and a bicarbonate.
(6d) The method according to (4d), wherein the organic salt is selected from the group consisting of a tartrate, an acetate, a citrate, an amino acid salt, an alginate, and a sorbate.
(7d) The method according to any one of (2d) to (5d), wherein the salt is selected from the group consisting of calcium chloride, sodium chloride, potassium chloride, sodium sulfate, calcium sulfate, calcium dihydrogen phosphate, sodium hydroxide, calcium hydroxide, and sodium bicarbonate.
(8d) The method according to any one of (1d) to (7d), wherein the pharmaceutical composition further contains a surfactant.
(9d) The method according to (8d), wherein the surfactant is an anionic surfactant.
(10d) The method according to (9d), wherein the anionic surfactant is sodium lauryl sulfate.
(11d) The method according to any one of (1d) to (10d), wherein the pharmaceutical composition further contains a pH adjusting agent.
(11d1) The method according to (11d), wherein the pH adjusting agent is selected from the group consisting of tartaric acid, citric acid, and maleic acid.
(12d) The method according to (11d) or (11d1), wherein the pH adjusting agent is tartaric acid.
(13d) The method according to any one of (1d) to (12d), wherein the pharmaceutical composition further contains one or more substances selected from a disintegrant, an excipient, and a binder.
(13d1) The method according to (12c), wherein the disintegrant is selected from the group consisting of carmellose calcium, crospovidone, sodium starch glycolate and croscarmellose sodium; the excipient is selected from the group consisting of starch, lactose hydrate and crystalline cellulose; and the binder is selected from the group consisting of hydroxypropyl cellulose, hydroxypropyl methyl cellulose, methyl cellulose, povidone (polyvinylpyrrolidone) and gum arabic powder.
(13d2) The method according to (13d), wherein the pharmaceutical composition contains one or more substances selected from crospovidone as the disintegrant, lactose hydrate as the excipient, and hydroxypropyl cellulose as the binder.
(14d) The method according to any one of (1d) to (13d2), wherein a weight ratio of the alectinib or a salt thereof and the inhibitor for gel-like substances is from 100 : 10 to 100 : 70 in terms of free form.
(14d1) The syrup according to any one of (1c) to (14d), wherein a weight ratio of the alectinib or a salt thereof and the surfactant and the inhibitor for gel-like substances is from 100 : 3 : 30 to 100 : 50 : 90 in terms of free form.
(15d) The method according to any one of (1d) to (14d1), wherein the pharmaceutical composition further contains one or more substances selected from a preservative, a thickener, a sweetener, and a flavor.
(16d) The method according to (14c), wherein the thickener is selected from the group consisting of xanthan gum, hydroxyethyl cellulose, and sodium carboxymethyl cellulose; the sweetener is selected from the group consisting of sucrose, mannitol, fructose, lactose, sodium saccharin, aspartame, xylitol, erythritol, sucralose, and acesulfame potassium; and the flavor is selected from the group consisting of a strawberry flavor, a raspberry flavor, an orange flavor, an apple flavor, a cherry black flavor, and a yogurt flavor.
(17d) The method according to any one of (1d) to (16d), wherein the pharmaceutical composition is a dry syrup.
(18d) The method according to any one of (1d) to (17d), wherein the pharmaceutical composition does not result in a decrease in fluidity when suspended.

The present invention can provide a syrup containing alectinib or a salt thereof as an active ingredient, where the decrease in fluidity when preparing or taking the syrup is prevented, and where the syrup has good fluidity and/or palatability. The present invention can also provide a syrup which has a good plasma concentration profile or is biologically equivalent. Alternatively, the present invention can provide a syrup having enhanced oral bioavailability compared to the capsule containing the same amount of alectinib or a salt thereof as the syrup.

### [Brief Description of Drawings]

[Figure 1] Figure 1 is a photograph showing the state of gel-like substance formation of dence Example 1.
[Figure 2] Figure 2 is a graph showing the results of taste evaluation in human.
[Figure 3] Figure 3 is a photograph showing the appearance of suspension of Examples 30 and 31.
[Figure 4] Figure 4 is a graph showing the dissolution profiles of Examples 30 and 31.
[Figure 5] Figure 5 is a graph showing the changes of plasma alectinib concentrations when the capsule and dry syrup are administered in each single dose.

### [Description of Embodiments]

Hereinafter, the present invention is described in detail.

"Alectinib" means a compound represented by formula (I): 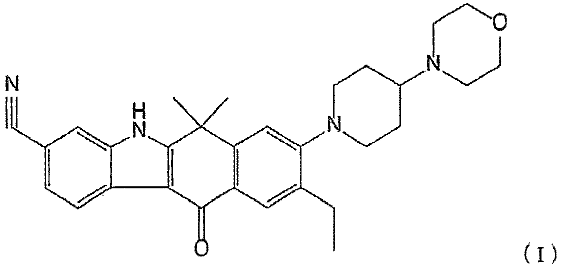 with the compound name: 9-ethyl-6,6-dimethyl-8-(4-morpholine-4-yl-piperidine-1-yl)-11-oxo-6,11-dihydro-5H-benzo[b]carbazole-3-carbonitrile.

The "salt" of alectinib is preferably a pharmaceutically acceptable salt, and examples of "pharmaceutically acceptable salt" include a hydrochloride, a hydrobromide, a hydroiodide, a phosphate, a phosphonate, a sulfate, a sulfonate such as a methanesulfonate or a p-toluenesulfonate, a carboxylate such as an acetate, a citrate, a malate, a tartrate, a succinate, or a salicylate, an alkali metal salt such as a sodium salt or a potassium salt; an alkaline earth metal salt such as a magnesium salt or a calcium salt; and an ammonium salt such as an ammonium salt, an alkylammonium salt, a dialkylammonium salt, a trialkylammonium salt, and a tetraalkylammonium salt.

The salt is preferably a hydrochloride, and most preferably a monohydrochloride.

Alectinib or a salt thereof can be produced by a known method (such as the method described in Patent Literature 2).

The monohydrochloride of alectinib may be amorphous or crystalline. When it is crystalline, a crystal having peaks at the diffraction angles (2θ) near 8.4°, 14.0°, 16.7°, 18.8° and 23.3° in the powder X-ray diffraction pattern is preferred. The amorphous of the monohydrochloride of alectinib can be produced according to the method described in WO2016/021707, and the crystal with these peaks can be produced according to the method described in WO2015/163447.

Alectinib or the salt thereof is contained in an amount of 10 to 60% by weight, preferably 15 to 40% by weight, and still more preferably 25 to 30% by weight, in terms of free form, based on the whole amount of the composition or preparation of the present invention.

"Surfactant" means a substance having both a hydrophilic group and a hydrophobic group in a molecule. The surfactant includes ionic surfactants and non-ionic surfactants.

An ionic surfactant means an ionic surfactant that, when dissolved in water, is ionized to become an ion (an atom or a group of atoms having a charge). The ionic surfactant is further classified into an anionic surfactant, a cationic surfactant, and an amphoteric surfactant, depending on the charge of the ion generated. Preferably, the ionic surfactant is an anionic surfactant.

Examples of the anionic surfactant include a sulfate-type surfactant such as alkyl sulfate (C12-18, Na, NH4, alkanolamine), POE alkylether sulfate (C12-18, Na, NH4, alkanolamine), POE alkylphenylether sulfate (C12-18, NH4, alkanolamine, Ca), POE benzyl(or stylyl)phenyl(or phenylphenyl)ether sulfate (Na, NH4, alkanolamine), polyoxyethylene, or polyoxypropylene block polymer sulfate (Na, NH4, alkanolamine); a sulfonate-type surfactant such as paraffin (alkane) sulfonate (C12-22, Na, Ca, alkanolamine), AOS (C14-16, Na, alkanolamine), dialkylsulfosuccinate (C8-12, Na, Ca, Mg), alkylbenzene sulfonate (C12, Na, Ca, Mg, NH4, alkylamine, alkanol, amine, cyclohexylamine), mono or dialkyl(C3-6)naphthalenesulfonate (Na, NH4, alkanolamine, Ca, Mg), naphthalenesulfonate-formaline condensate (Na, NH4), alkyl(C8-12)diphenylether disulfonate (Na, NH4), lignin sulfonate (Na, Ca), POE alkyl(C8-12)phenylether sulfonate (Na), or half-ester of POE alkyl(C12-18) ether sulfosuccinic acid (Na); a carboxylic acid-type surfactant such as a fatty acid salt (C12-18, Na, K, NH4, alkanolamine), N-methyl-fatty acid sarcosinate (C12-18, Na), or a resin acid salt (Na, K); and a phosphate-type surfactant such as POE alkyl(C12-18)ether phosphate (Na, alkanolamine), POE mono or dialkyl(C8-12) phenyl ether phosphate (Na, alkanolamine), POE benzyl(or stylyl)phenyl(or phenylphenyl)ether phosphate (Na, alkanolamine), polyoxyethylene-polyoxypropylene block polymer (Na, alkanolamine), phosphatidylcholine-phosphatidylethanolimine (lecithin), or alkyl(C8-12) phosphate. Preferred examples of the surfactant include monoalkyl sulfates such as sodium lauryl sulfate, sodium tetradecyl sulfate, sodium hexadecyl sulfate, and sodium octadecyl sulfate, dioctyl sodium sulfosuccinate, sodium lauroyl sarcosinate, and sodium dodecylbenzenesulfonate. The most preferred surfactant is sodium lauryl sulfate.

In the present invention, two or more surfactants may be used in combination in appropriate proportions.

When sodium lauryl sulfate is used in the present invention, the crystal thereof obtained by spray drying or crystallization can be used. As crystalline polymorphs of sodium lauryl sulfate, monohydrate, 1/2-hydrate, 1/8-hydrate, and ansolvate are known (Journal of Crystal Growth 263 (2004) 480-490), and any of these crystals can be used in the composition or preparation of the present invention.

The amount of the surfactant is 2.5% or more by weight, preferably 2.5% or more by weight and 20% or less by weight, more preferably 2.5% or more by weight and 15% or less by weight, and most preferably 3.75% or more by weight and 7.5% or less by weight, based on the whole amount of the preparation.

Regarding the surfactant contained in the composition or preparation of the present invention, the weight ratio of alectinib or a salt thereof and the surfactant is preferably from 100 : 3 to 100 : 50, more preferably from 100 : 12.5 to 100 : 50, and most preferably from 100 : 12.5 to 100 : 25.

"Inhibitor for gel-like substances" means a substance capable of suppressing the formation of a gel-like substance that occurs when a preparation containing a composition containing alectinib or a salt thereof and a surfactant is suspended, by allowing the substance to be present together in the composition. The gel-like or the formation of a gel-like substance refers to a significant decrease in fluidity of the suspension due to the interaction between alectinib or a salt thereof and the anionic surfactant sodium lauryl sulfate, or a formation of a substance that significantly decreases the fluidity of the suspension. In addition, a decrease in fluidity refers to a state in which the fluidity is not present even when the suspension is shaken, and the solidification to a degree that is inappropriate for dispensation or administration is observed. Specifically, for a suspension of a composition containing alectinib or a salt thereof and an anionic surfactant such as sodium lauryl sulfate, a decrease in fluidity refers to the fluidity of the suspension that is decreased compared to that of a suspension similarly prepared with a composition containing alectinib or a salt thereof but not sodium lauryl sulfate. More specifically, a significant decrease in fluidity occurs when conventional granules used in a capsule containing alectinib or a salt thereof and an anionic surfactant such as sodium lauryl sulfate are administered to a child by suspending the granule (for example, 2000 mg of granule containing 600 mg of alectinib in terms of free form) in a small amount of water (for example, 10 ml), causing alectinib or a salt thereof and an anionic surfactant to form a complex and form a gel-like substance. When the inhibitor for gel-like substances is allowed to be present together in a composition containing alectinib or a salt thereof and an anionic surfactant such as sodium lauryl sulfate, a common ionic effect or ion exchange can reduce the solubility of alectinib or a salt thereof or the solubility of anionic surfactant, and suppress the formation of a gel-like substance between alectinib and the anionic surfactant, thereby suppressing the decrease in fluidity occurring when suspended. As the inhibitor for gel-like substances, a salt can be used. As the inhibitor for gel-like substances, one kind may be used alone, or two or more kinds may be used in combination.

In the present invention, "salt" refers to a salt including monovalent to trivalent inorganic or organic salts. Preferably, the salt is a water-soluble salt. "Water-soluble salt" as used herein refers to a salt having a solubility of 0.1 g or more, preferably 1 g or more, more preferably 3 g or more, further preferably 10 g or more, in 100 g of water at 20°C.

As the salt, one kind may be used alone, or two or more kinds of these salts may be used in combination as appropriate.

"Inorganic salt" as used herein refers to a compound in which an anion derived from an inorganic acid and a cation derived from an inorganic base are ionically bonded. Examples of the anion constituting the inorganic salt include a halide ion, a sulfate ion, a nitrate ion, a phosphate ion, a carbonate ion, a bicarbonate ion, and a hydroxide ion. Examples of the cation constituting the inorganic salt include a metal ion [e.g., an alkali metal ion (a sodium ion, a potassium ion, and the like), an alkaline earth metal ion (a calcium ion, a magnesium ion, and the like)], and an ammonia ion. Preferably, the inorganic salt is a water-soluble salt. The inorganic salt may form a hydrate.

Specific examples of the inorganic salt include an inorganic salt of a metal such as a halide salt, a carbonate or bicarbonate, a sulfate, a phosphate, or a hydroxide salt of a metal, and an inorganic salt of ammonia such as a halide salt, a carbonate or bicarbonate, a sulfate, a phosphate or a hydroxide salt of ammonia. Preferably, the inorganic salt is an inorganic salt of a metal, and further preferably, it is an inorganic salt of an alkali or alkaline earth metal (an inorganic salt of sodium, an inorganic salt of potassium, an inorganic salt of calcium, or an inorganic salt of magnesium).

Examples of the halide salt include a halide salt of a metal, such as a halide of an alkali or alkaline earth metal, such as sodium chloride, potassium chloride, calcium chloride or a monohydrate, dihydrate, tetrahydrate or hexahydrate thereof, or magnesium chloride or a hexahydrate thereof; and a halide salt of ammonia, such as ammonium chloride. Preferably, the halide salt is a chloride salt.

Examples of the carbonate or bicarbonate include a carbonate or hydrogen carbonate of an alkali or alkaline earth metal, such as sodium carbonate or a monohydrate or decahydrate thereof, sodium bicarbonate, calcium carbonate; and a carbonate of ammonia, such as ammonium carbonate, or a hydrate thereof.

Examples of the sulfate include a sulfate of an alkali or alkaline earth metal, such as sodium sulfate or decahydrate thereof, magnesium sulfate or a heptahydrate thereof, calcium sulfate or a hemihydrate or dihydrate thereof, and a sulfate of ammonia, such as ammonium sulfate, or a hydrate thereof.

Examples of the phosphate include a phosphate of an alkali or alkaline earth metal, such as sodium phosphate, calcium dihydrogen phosphate, calcium monohydrogen phosphate, tricalcium phosphate, and hydrates of these; and a phosphate of ammonia, such as ammonium phosphate, or a hydrate thereof.

The hydroxide salt refers to a hydroxide salt of an alkali or alkaline earth metal, and examples thereof include a hydroxide salt of a metal such as sodium hydroxide, potassium hydroxide, magnesium hydroxide, and calcium hydroxide. The hydroxide salt further includes a hydroxide salt of ammonia, such as ammonium hydroxide, or a hydrate thereof. Sodium hydroxide and calcium hydroxide are preferred.

The inorganic salt is preferably calcium chloride, sodium chloride, potassium chloride, sodium sulfate, calcium sulfate, sodium bicarbonate, calcium dihydrogen phosphate, or hydrates of these. The inorganic salt is, further preferably, calcium chloride, potassium chloride, sodium sulfate, calcium sulfate, calcium dihydrogen phosphate, or hydrates of these.

A neutral inorganic salt means a normal salt consisting of a strong acid and a strong base, and specific examples thereof include metal halides such as sodium chloride, potassium chloride, calcium chloride, sodium sulfate, calcium sulfate, and hydrates of these.

The inorganic salt described above may be synthesized by known methods or may be obtained commercially. As the inorganic salt in the present invention, one kind may be used alone, or two or more kinds may be used in any combination.

"Organic salt" as used herein refers to a compound in which an anion derived from an organic acid and a cation derived from the aforementioned inorganic base are ionically bonded at 1 : 1. Examples of the anion constituting the organic acid include a citrate ion, an acetate ion, a tartrate ion, an amino acid ion, an alginate ion, and a sorbate ion. Specific examples of the organic salt include an organic salt of a metal, such as a citrate, an acetate, a tartrate, an amino acid salt, an alginate, or a sorbate of a metal; and an organic salt of ammonia such as a citrate, an acetate, a tartrate, an amino acid salt, an alginate, or a sorbate of ammonia. Preferably, the organic salt is an organic salt of metal, and further preferably, it is an organic salt of an alkali or alkaline earth metal (an organic salt of sodium, an organic salt of potassium, an organic salt of calcium, or an organic salt of magnesium).

Examples of the citrate include a citrate of a metal, such as monosodium citrate, disodium citrate, or calcium citrate; and a citrate of ammonia such as ammonium citrate. Examples of the acetate include an acetate of a metal such as sodium acetate, potassium acetate or magnesium acetate, and an acetate of ammonia such as ammonium acetate. Examples of the tartrate include a tartrate of a metal, such as sodium tartrate; and a tartrate of ammonia such as ammonium tartrate. Examples of the amino acid salt include an amino acid salt of a metal, such as sodium glutamate, magnesium aspartate; and an amino acid salt of ammonia such as ammonium glutamate. Examples of the alginate include sodium alginate and ammonium alginate, and examples of the sorbate include potassium sorbate and ammonium sorbate. These organic salts also include their hydrates (such as sodium acetate trihydrate, magnesium acetate tetrahydrate), and the like. Preferably, the organic salt is an organic salt of an alkali or an alkaline earth metal salt selected from sodium tartrate, sodium glutamate, magnesium aspartate, sodium alginate, and potassium sorbate.

The organic salt described above may be synthesized by known methods or may be obtained commercially. As the organic salt in the present invention, one kind may be used alone, or two or more kinds may be used in any combination.

The most preferred inhibitor for gel-like substances is calcium chloride or a hydrate thereof.

The amount of the inhibitor for gel-like substances is 10% or more by weight, 10% or more by weight and 30% or less by weight, 20% or more by weight, or 20% or more by weight and 30% or less by weight, based on the whole amount of the syrup.

The weight ratio of alectinib or a salt thereof and the inhibitor for gel-like substances is preferably from 100 : 10 to 100 : 70, more preferably from 100 : 30 to 100 : 70, and most preferably from 100 : 60 to 100 : 70 in terms of free form.

In the present invention, "capsule containing the same amount of alectinib or a salt thereof as the syrup" refers to a capsule that is an approved capsule containing alectinib hydrochloride (e.g., ALECENSA(R) capsule 150 mg, CHUGAI PHARMACEUTICAL CO., LTD.) or a generic drug thereof, and is adjusted in the number or amount to contain the same amount (in terms of free form) as the syrup of the present invention. Specifically, a capsule containing the same amount of alectinib or a salt thereof as a syrup containing 600 mg (in terms of free form) of alectinib or a salt thereof means four capsules containing 150 mg (in terms of free form) of alectinib hydrochloride.

"Plasma concentration profile" in the present invention means an indicator related to the change of the average plasma concentration of alectinib (free form) when a preparation containing alectinib or a salt thereof is orally administered to a healthy adult in a single dose. The plasma concentration of alectinib and its M4 metabolite in a venous blood sample is measured by LC-MS/MS method and analyzed according to the method (Method 2) described in Bioanalysis (2016) 8(14), 1465-79.

"Plasma concentration profile equivalent to or greater than" in the present invention refers to the plasma concentration profile of a subject preparation equivalent to or greater than that of a comparative preparation. Specifically, it refers to the change of the average plasma concentration of alectinib (free form) when a syrup preparation containing alectinib or a salt thereof is orally administered to a healthy adult in a single dose which is equivalent or increased compared to that when a control capsule containing the same amount of alectinib or a salt thereof as the syrup preparation is orally administered.

"Bioequivalence" in the present invention refers to the bioavailability of a subject preparation equivalent to that of a comparative preparation. In the single dose study, AUC and Cₘₐₓ are taken as bioequivalence determination parameters, where Cₘₐₓ is given by the measured value, and AUC is given by the value calculated by trapezoidal method. Specifically, the average plasma concentration of alectinib (free form) and AUC when a syrup preparation containing alectinib or a salt thereof is orally administered to a healthy adult in a single dose are compared to those when a control capsule containing the same amount of alectinib or a salt thereof as the syrup preparation is orally administered. Generally, when the 90% confidence interval of the difference in the average logarithmic value of the bioequivalence determination parameter of the test preparation and the comparative preparation is in the range of log(0.80) to log(1.25), the test preparation and the comparative preparation are determined to be biologically equivalent. Herein, in the comparison by the geometric mean value of measured values, an case of 80% or more and 125% or less relative to the value of the parameter of the capsule set to 100% is taken as equivalent.

"Enhanced oral bioavailability" in the present invention refers to the bioavailability of a subject preparation that is enhanced compared to that of the comparative preparation. Herein, in the comparison by the geometric mean value of measured values, an case of over 125% relative to the value of the parameter of the capsule set to 100% is taken as enhanced.

"Pharmaceutical composition" in the present invention means a mixture of two or more substances used in the treatment and prevention and the like of a disease. In one aspect of the present invention, the pharmaceutical composition is used in the production of a pharmaceutical preparation. "Pharmaceutical preparation" means a preparation for the treatment or prevention of a disease, and in the present invention, a syrup is preferred. "Syrup" is a viscous liquid or solid preparation containing saccharides or a sweetener (preparation for syrup) that can be administered orally, wherein the active ingredient is mainly absorbed from the intestinal tract. "Syrup" refers to the syrup prescribed in the Japanese Pharmacopoeia, 16th Edition, General Rules for Preparations, and includes a dry syrup. "Preparation for syrup" is a granular or powdered preparation that becomes a syrup when water is added. It is also referred to as dry syrup. The preparation for syrup, or dry syrup is used by dissolving or suspending a granular or powdered preparation with water or the like, when used.

Examples of "excipient" include starches such as corn starch, potato starch, wheat starch, rice starch, partially pregelatinized starch, pregelatinized starch, and porous starch; sugars or sugar alcohols such as lactose hydrate, fructose, glucose, mannitol, and sorbitol; crystalline cellulose, precipitated calcium carbonate, and calcium silicate. Preferred examples of the excipient include starches such as starch, potato starch, and corn starch, lactose hydrate, and crystalline cellulose. The most preferred excipient is lactose hydrate.

"Disintegrant" in the present invention is an ingredient for promoting the rapid disintegration of a solid preparation or a preparation for syrup when a solid preparation such as a tablet is taken orally, or when a preparation for syrup or the like is suspended or dissolved in water or the like.

Examples of the disintegrant include sodium starch glycolate, low-substituted hydroxypropyl cellulose, calcium carmellose, pregelatinized starch, corn starch, sodium croscarmellose, crystalline cellulose, anhydrous silicic acid, and carmellose. Preferably the disintegrant is selected from carmellose calcium, crospovidone, sodium starch glycolate, and croscarmellose sodium, and most preferably the disintegrant is crospovidone.

The amount of the disintegrant used is, for example, 5% or more by weight, preferably 7.5% or more by weight, and still more preferably 10% or more by weight, based on the whole amount of the composition or preparation of the present invention. The upper limit of the amount used is not particularly limited, but is, for example, 30% by weight.

Examples of "binder" include polyvinylpyrrolidone, macrogol, and the same compounds as the excipient described above. Specific examples of the binder include hydroxypropyl cellulose, hydroxypropyl methyl cellulose, methyl cellulose, povidone (polyvinylpyrrolidone), and gum arabic powder. The most preferred binder is hydroxypropyl cellulose. The amount of the binder used is preferably 0.1% or more by weight, and still more preferably 0.5% or more by weight, based on the whole amount of the composition or preparation of the present invention. The upper limit of the amount used is not particularly limited, but is, for example, 40% by weight.

Examples of "emulsifier" include polysorbate 80, polyoxyl 40 stearate, and lauromacrogol.

"Colorant" may be any colorant as long as it is permitted to add to medicaments, and examples thereof include food dyes such as food yellow No. 5 (Sunset Yellow, U.S. food yellow No. 6), food red No. 2, and food blue No. 2, lake food colors, and iron sesquioxide.

"Fluidizer" is used to improve the fluidity of mixed powder or granules, and typical examples thereof include talc, and light anhydrous silicic acid and hydrated silicon dioxide, which are silicon dioxides. Here, light anhydrous silicic acid may be any compound composed mainly of hydrated silicon dioxide (SiO2nH2O), where n indicates an integer, and specific examples thereof include SYLYSIA 320 (trade name, Fuji Silysia Chemical Ltd.) and AEROSIL 200 (trade name, NIPPON AEROSIL Co., Ltd.).

Suitable examples of "preservative" include sodium benzoate, paraoxybenzoic acid esters, chlorobutanol, benzyl alcohol, phenethyl alcohol, dehydroacetic acid, sorbic acid, and potassium sorbate.

Suitable examples of "antioxidant" include sulfite and ascorbic acid.

Two or more of the above additives may be used by mixing them in appropriate proportions.

Ethanol, phenol, chlorocresol, purified water, distilled water, and the like can be used as a solvent for producing a liquid preparation.

The syrup of the present invention can be produced by a general method after mixing alectinib or a salt thereof with a surfactant, an inhibitor for gel-like substances, and an additive described above. Preferably, it is produced according to the following production method.
1) Alectinib or a salt thereof is mixed with an additive such as a surfactant, an inhibitor for gel-like substances, an excipient, a disintegrant, a binder, or a sweetener, and then the mixture is granulated with addition or spraying of a solvent (e.g., purified water, ethanol, or a mixture thereof). To the obtained granules, an appropriate amount of a flavor, a thickener, and the like are added and mixed to prepare the syrup of the present invention.
2) Alectinib or a salt thereof is mixed with an additive such as a surfactant, an inhibitor for gel-like substances, an excipient, a disintegrant, or a sweetener, and then the mixture is granulated with addition or spraying of a solvent obtained by dispersing or dissolving a binder and, if necessary, other additives in a solvent (e.g., purified water, ethanol, or a mixture thereof). To the obtained granules, an appropriate amount of a flavor, a thickener, and the like are added and mixed to prepare the syrup of the present invention.

A dry syrup is a granule or a preparation obtained by adding an extra-granules to a granule, and is generally produced according to the granule preparation method using saccharides or a sweetener.

The present invention also relates to a pharmaceutical composition containing (i) alectinib or a salt thereof, (ii) a surfactant, and (iii) an inhibitor for gel-like substances.

One aspect of the pharmaceutical composition of the present invention also relates to a pharmaceutical composition containing (i) alectinib or a salt thereof, (ii) a surfactant, (iii) an inhibitor for gel-like substances, and (iv) a thickener, a sweetener, a taste masking agent, an odor masking agent, or a flavor.

Another aspect of the pharmaceutical composition of the present invention also relates to a dry syrup containing (i) a granule containing alectinib or a salt thereof, (ii) a surfactant, and (iii) an inhibitor for gel-like substances.

Further aspect of the pharmaceutical composition of the present invention is a dry syrup containing (i) alectinib or a salt thereof, (ii) a surfactant, (iii) an inhibitor for gel-like substances, and (iv) a thickener, a sweetener, a taste masking agent, an odor masking agent, or a flavor.

Still further aspect of the pharmaceutical composition of the present invention is a suspension agent containing (i) alectinib or a salt thereof, (ii) a surfactant, (iii) an inhibitor for gel-like substances, and (iv) a thickener, a sweetener, a taste masking agent, an odor masking agent, or a flavor.

In the present invention, "granules" mean particles of nearly uniform shape and size, and are obtained by granulating a raw material such as powder, lump, solution, or molten liquid form by wet granulation, dry granulation, heated granulation, or the like. "Non-granular" means a state in which no granule is formed.

In the present invention, the dry syrup or granule may contain a variety of additives in addition to the surfactant or disintegrant described above.

For example, the dry syrup or granule may contain alectinib or a salt thereof, a disintegrant, a surfactant, an excipient, and a binder. The dry syrup or granule may further contain one or more additives selected from a lubricant, a coating agent, a stabilizer, a thickener, a sweetener, a taste masking agent, an odor masking agent, or a flavor, and a diluent. Such a dry syrup or granule can be produced by granulating a composition containing the compound represented by formula (I) or a salt thereof, and optionally additives such as a disintegrant, a surfactant, an excipient, a lubricant, a coating agent, a binder, a stabilizer, a thickener, a sweetener, a taste masking agent, an odor masking agent, or a flavor, or a diluent, by a conventional granulation process.

In the present invention, "Extra-granules" or "Extra-component" refers to a component added to the outside of the granule in a dry syrup. As the extra-granules, an additive such as a disintegrant, a lubricant, a fluidizer, a thickener, a sweetener, a taste masking agent, an odor masking agent or a flavor, or a thickener can also be added.

The disintegrant may be contained in the granule or in the extra-granules of the dry syrup. Preferably, the disintegrant is selected from sodium starch glycolate, low-substituted hydroxypropyl cellulose, carmellose calcium, pregelatinized starch, corn starch, croscarmellose sodium, crystalline cellulose, anhydrous silicic acid, and carmellose, more preferably selected from carmellose calcium, crospovidone, sodium starch glycolate and croscarmellose sodium, and most preferably crospovidone.

The dry syrup of the present invention may contain an inhibitor for gel-like substances in a granule, and then the dry syrup may be a dry syrup containing a granule containing (i) alectinib or a salt thereof, and an inhibitor for gel-like substances, and (ii) a surfactant. The inhibitor for gel-like substances is more preferably present together as an intragranular component.

The pharmaceutical composition of the present invention may contain, in addition to the surfactant, a dissolution aid such as the organic polymer described below.

Examples of "dissolution aid" include an organic polymer. Specific examples of the "organic polymer" used in the present invention include polysaccharides such as hydroxypropyl cellulose (hereinafter also referred to as HPC), hydroxypropyl methyl cellulose, methyl cellulose, propylene glycol alginate ester, agar powder, guar gum, zein, or hydroxyethyl methyl cellulose; a synthetic resin such as carboxyvinyl polymer, polyvinyl alcohol, or vinyl acetate resin, or sodium polystyrene sulfonate; and a phosphoprotein such as casein or casein sodium.

Among the organic polymers, a polymer having a solubility of 1 g/100 g or more in water is referred to as a water-soluble polymer. Specific examples of the water-soluble polymer include hydroxypropyl cellulose, hydroxypropyl methyl cellulose, methylcellulose, propylene glycol alginate ester, casein sodium, a carboxyvinyl polymer, agar powder, guar gum, copolyvidon, hydroxyethyl methyl cellulose, and polyvinyl alcohol.

Among the organic polymers, a polymer that dissolves under acidic conditions of pH 1.2 to 3.5 that is the pH of the gastric juice is referred to as a gastric soluble polymer, and a polymer that dissolves quickly in pH 6 to 8 of the intestinal tract is referred to as an enteric soluble polymer. Examples of the gastric soluble polymer include aminoalkyl methacrylate copolymer E or polyvinylacetal diethylaminoacetate. Examples of the enteric soluble polymer include methacrylic acid copolymer LD (emulsion), methacrylic acid copolymer S, purified shellac, carboxymethylethyl cellulose, cellulose acetate phthalate (cellacefate), hydroxypropyl methyl cellulose acetate succinate, casein, and zein.

Preferred examples of the dissolution aid include casein, casein sodium, skim milk powder, dioctyl sodium sulfosuccinate, polyoxyl 40 stearate, sorbitan trioleate, polyoxyethylene (105) polyoxypropylene (5) glycol, polyoxyethylene cured castor oil 60, polyoxyl 35 castor oil, lauromacrogol, lauroyl sarcosine sodium, sodium tetradecyl sulfate, sodium hexadecyl sulfate, sodium octadecyl sulfate, sodium methyl sulfate, sodium ethyl sulfate, sodium butyl sulfate, sodium octyl sulfate, sodium decyl sulfate, and sodium dodecyl benzene sulfonate.

The dissolution aid may be granular or obtained by spray drying.

Examples of the excipient that can be included in the granule of the dry syrup according to the present invention include starches such as corn starch, potato starch, wheat starch, rice starch, partially pregelatinized starch, pregelatinized starch, and porous starch; sugars or sugar alcohols such as lactose hydrate, fructose, glucose, mannitol, and sorbitol; crystalline cellulose, precipitated calcium carbonate, and calcium silicate. Preferred examples of the excipient include starches such as starch, potato starch, and corn starch, lactose hydrate, and crystalline cellulose, and the lactose hydrate is further preferred. The amount of the excipient used is preferably 5 to 60% by weight, and still more preferably 5 to 20% by weight, based on 100% by weight of the composition or preparation.

Examples of the binder that can be contained in the granules of the dry syrup according to the present invention include hydroxypropyl cellulose, polyvinylpyrrolidone, macrogol, and the same compounds as the excipients described above. Specific examples of the binder include hydroxypropyl cellulose, hydroxypropyl methyl cellulose, methyl cellulose, povidone (polyvinylpyrrolidone), and gum arabic powder, and preferably include hydroxypropyl cellulose. The amount of the binder used is preferably 0.1 to 50% by weight, more preferably 0.5 to 40% by weight, and still more preferably 0.5 to 10% by weight, based on 100% by weight of the composition or preparation.

Examples of the lubricant in the present invention include magnesium stearate, calcium stearate, talc, sucrose fatty acid esters, and sodium stearyl fumarate.

Examples of the preservative in the present invention include sodium benzoate, paraoxybenzoic acid esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid or potassium sorbate. The preservative is preferably sodium benzoate and potassium sorbate. The most preferred preservative is sodium benzoate.

The syrup of the present invention can include a thickener, a sweetener, a taste masking agent, an odor masking agent, a flavor, a pH adjusting agent, and the like, which are commonly used in pediatric preparations. These can be used as an intragranular component or an extra-granules in the dry syrup. Preferably, at least one or two of the thickener, the sweetener, the taste masking agent, the odor masking agent, and the flavor are used as the extra-granules.

"Thickener" is used to evenly disperse suspension components in a solution during production of a syrup or suspension of a dry syrup. Specific examples of the thickener include xanthan gum, hydroxyethyl cellulose, and sodium carboxymethyl cellulose. The thickener is preferably xanthan gum or sodium carboxymethyl cellulose. The most preferred thickener is xanthan gum.

"Sweetener" is used to sweeten medicines. Examples of the sweetener include natural saccharides such as sucrose, mannitol, fructose, and lactose, and licorice powders, and artificial sweeteners such as saccharin sodium, aspartame, xylitol, erythritol, sucralose, and acesulfame potassium. The sweetener is preferably saccharin sodium, sucralose, or acesulfame potassium. The most preferred sweetener is sucralose.

"Taste masking agent" is used to add to a bitter drug to make it easy to drink. As the taste masking agent, the saccharides described above are mainly used.

"Odor masking agent" is used to eliminate or soften the unpleasant odor of medicines. As the odor masking agent, l-menthol, peppermint oil, and the like are used.

Examples of "flavor" include a strawberry flavor, a raspberry flavor, an orange flavor, an apple flavor, a cherry black flavor, and a yogurt flavor. The flavor is preferably a strawberry flavor.

Examples of "pH adjusting agent" include hydrochloric acid, citric acid, tartaric acid, acetic acid, maleic acid, and trisodium citrate. The "pH adjusting agent" is preferably tartaric acid, citric acid, and maleic acid. The most preferred pH adjusting agent is tartaric acid.

The present invention also includes:
a preparation for syrup, containing a composition consisting of alectinib or a salt thereof, an anionic surfactant, and an inhibitor for gel-like substances, wherein the preparation has a better dissolution property of the agent compared to the composition that does not contain the inhibitor for gel-like substances;
the preparation for syrup, which is granular; and
the preparation for syrup, which is a dry syrup;
a syrup consisting of alectinib or a salt thereof, an anionic surfactant, and an inhibitor for gel-like substances, wherein the syrup has a better fluidity and/or palatability compared to the composition that does not contain the inhibitor for gel-like substances.

The present invention further includes:
a method of decreasing the fluidity in suspension of a composition containing alectinib or a salt thereof and an anionic surfactant by allowing an inhibitor for gel-like substances to be present together in the composition, compared to the composition that does not contain the inhibitor for gel-like substances;
a method of improving the fluidity and/or palatability of a composition containing alectinib or a salt thereof and an anionic surfactant by allowing an inhibitor for gel-like substances to be present together in the composition, compared to the composition that does not contain the inhibitor for gel-like substances; and
a method for producing a preparation for syrup, the method including a step of allowing an inhibitor for gel-like substances to be present together in the composition containing alectinib or a salt thereof and an anionic surfactant, wherein the preparation has a better dissolution property of an agent compared to the composition that does not contain the inhibitor for gel-like substances.

Specific examples of the syrup according to the present invention include:
a syrup or dry syrup containing alectinib or a salt thereof; a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, acetic acid, maleic acid, and trisodium citrate; a preservative selected from sodium benzoate, paraoxybenzoic acid esters, chlorobutanol, benzyl alcohol, phenethyl alcohol, dehydroacetic acid, sorbic acid, and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant selected from the group consisting of carmellose calcium, crospovidone, sodium starch glycolate, and sodium croscarmellose; a binder selected from the group consisting of hydroxypropyl cellulose, hydroxypropyl methyl cellulose, methyl cellulose, povidone (polyvinylpyrrolidone), and gum arabic powder; a sweetener selected from the group consisting of sucrose, mannitol, fructose, lactose, sodium saccharin, aspartame, xylitol, erythritol, sucralose, and acesulfame potassium; and a flavor selected from the group consisting of a strawberry flavor, a raspberry flavor, an orange flavor, an apple flavor, a cherry black flavor, and a yogurt flavor;
a syrup or dry syrup containing alectinib or a salt thereof a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, and maleic acid; a preservative selected from sodium benzoate and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant that is crospovidone; a binder that is hydroxypropyl cellulose; a sweetener selected from the group consisting of sodium saccharin, sucralose, and acesulfame potassium; and a flavor selected from the group consisting of a strawberry flavor, a raspberry flavor, an orange flavor, and a cherry black flavor;
a syrup or a dry syrup containing alectinib or a salt thereof a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, acetic acid, maleic acid, and trisodium citrate; a preservative selected from sodium benzoate, paraoxybenzoic acid esters, chlorobutanol, benzyl alcohol, phenethyl alcohol, dehydroacetic acid, sorbic acid, and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant selected from the group consisting of carmellose calcium, crospovidone, sodium starch glycolate, and sodium croscarmellose; a binder selected from the group consisting of hydroxypropyl cellulose, hydroxypropyl methyl cellulose, methyl cellulose, povidone (polyvinylpyrrolidone), and gum arabic powder; a sweetener selected from the group consisting of sucrose, mannitol, fructose, lactose, sodium saccharin, aspartame, xylitol, erythritol, sucralose, and acesulfame potassium; and a flavor selected from the group consisting of a strawberry flavor, a raspberry flavor, an orange flavor, an apple flavor, a cherry black flavor, and a yogurt flavor, wherein the syrup has an equivalent or greater plasma concentration profile relative to a capsule containing the same amount of alectinib or a salt thereof as the syrup;
a syrup or dry syrup containing alectinib or a salt thereof a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, and maleic acid; a preservative selected from sodium benzoate and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant that is crospovidone; a binder that is hydroxypropyl cellulose; a sweetener selected from the group consisting of sodium saccharin, sucralose, and acesulfame potassium; and a flavor selected from the group consisting of a strawberry flavor, a raspberry flavor, an orange flavor, and a cherry black flavor, wherein the syrup has a plasma concentration profile equivalent to or greater than a capsule containing the same amount of alectinib or a salt thereof as the syrup;
a syrup or dry syrup containing alectinib or a salt thereof; a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, and maleic acid; a preservative selected from sodium benzoate and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant that is crospovidone; a binder that is hydroxypropyl cellulose; a sweetener selected from the group consisting of sodium saccharin, sucralose, and acesulfame potassium; and a flavor selected from the group consisting of a strawberry flavor, a raspberry flavor, an orange flavor, and a cherry black flavor, wherein a weight ratio of the alectinib or a salt thereof and the inhibitor for gel-like substances is from 100 : 30 to 100 : 90 in terms of free form, and/or a weight ratio of the alectinib or a salt thereof and the surfactant and the inhibitor for gel-like substances is from 100 : 3 : 30 to 100 : 50 : 90 in terms of free form, and wherein the syrup has a plasma concentration profile equivalent to or greater than a capsule containing the same amount of alectinib or a salt thereof as the syrup;
a syrup or dry syrup containing alectinib or a salt thereof; a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, acetic acid, maleic acid, and trisodium citrate; a preservative selected from sodium benzoate, paraoxybenzoic acid esters, chlorobutanol, benzyl alcohol, phenethyl alcohol, dehydroacetic acid, sorbic acid, and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant selected from the group consisting of carmellose calcium, crospovidone, sodium starch glycolate, and sodium croscarmellose; a binder selected from the group consisting of hydroxypropyl cellulose, hydroxypropyl methyl cellulose, methyl cellulose, povidone (polyvinylpyrrolidone), and gum arabic powder; a sweetener selected from the group consisting of sucrose, mannitol, fructose, lactose, sodium saccharin, aspartame, xylitol, erythritol, sucralose, and acesulfame potassium; and a flavor selected from the group consisting of a strawberry flavor, a raspberry flavor, an orange flavor, an apple flavor, a cherry black flavor, and a yogurt flavor, wherein the syrup has one or two or more plasma concentration profiles selected from the group consisting of AUC and Cₘₐₓ equivalent to or greater than a capsule containing the same amount of alectinib or a salt thereof as the syrup;
a syrup or dry syrup containing alectinib or a salt thereof; a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, and maleic acid; a preservative selected from sodium benzoate and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant that is crospovidone; a binder that is hydroxypropyl cellulose; a sweetener selected from the group consisting of sodium saccharin, sucralose, and acesulfame potassium; and a flavor selected from the group consisting of a strawberry flavor, a raspberry flavor, an orange flavor, and a cherry black flavor, wherein the syrup has one or two or more plasma concentration profiles selected from the group consisting of AUC and Cₘₐₓ equivalent to or greater than a capsule containing the same amount of alectinib or a salt thereof as the syrup;
a syrup or dry syrup containing alectinib or a salt thereof a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, and maleic acid; a preservative selected from sodium benzoate and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant that is crospovidone; a binder that is hydroxypropyl cellulose; a sweetener selected from the group consisting of sodium saccharin, sucralose, and acesulfame potassium; and a flavor selected from the group consisting of a strawberry flavor, a raspberry flavor, an orange flavor, and a cherry black flavor, wherein a weight ratio of the alectinib or a salt thereof and the inhibitor for gel-like substances is from 100 : 30 to 100 : 90 in terms of free form, and/or a weight ratio of the alectinib or a salt thereof and the surfactant and the inhibitor for gel-like substances is from 100 : 3 : 30 to 100 : 50 : 90 in terms of free form, and wherein the syrup has one or two or more plasma concentration profiles selected from the group consisting of AUC and Cₘₐₓ equivalent to or greater than a capsule containing the same amount of alectinib or a salt thereof as the syrup;
a syrup or dry syrup containing alectinib or a salt thereof; a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, acetic acid, maleic acid, and trisodium citrate; a preservative selected from sodium benzoate, paraoxybenzoic acid esters, chlorobutanol, benzyl alcohol, phenethyl alcohol, dehydroacetic acid, sorbic acid, and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant selected from the group consisting of carmellose calcium, crospovidone, sodium starch glycolate, and sodium croscarmellose; a binder selected from the group consisting of hydroxypropyl cellulose, hydroxypropyl methyl cellulose, methyl cellulose, povidone (polyvinylpyrrolidone), and gum arabic powder; a sweetener selected from the group consisting of sucrose, mannitol, fructose, lactose, sodium saccharin, aspartame, xylitol, erythritol, sucralose, and acesulfame potassium; and a flavor selected from the group consisting of a strawberry flavor, a raspberry flavor, an orange flavor, an apple flavor, a cherry black flavor, and a yogurt flavor, wherein the syrup has AUC and Cₘₐₓ equivalent to or greater than a capsule containing the same amount of alectinib or a salt thereof as the syrup;
a syrup or dry syrup containing alectinib or a salt thereof a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, and maleic acid; a preservative selected from sodium benzoate and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant that is crospovidone; a binder that is hydroxypropyl cellulose; a sweetener selected from the group consisting of sodium saccharin, sucralose, and acesulfame potassium; and a flavor selected from the group consisting of a strawberry flavor, a raspberry flavor, an orange flavor, and a cherry black flavor, wherein the syrup has AUC and Cₘₐₓ equivalent to or greater than a capsule containing the same amount of alectinib or a salt thereof as the syrup;
a syrup or dry syrup containing alectinib or a salt thereof a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, and maleic acid; a preservative selected from sodium benzoate and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant that is crospovidone; a binder that is hydroxypropyl cellulose; a sweetener selected from the group consisting of sodium saccharin, sucralose, and acesulfame potassium; and a flavor selected from the group consisting of a strawberry flavor, a raspberry flavor, an orange flavor, and a cherry black flavor, wherein a weight ratio of the alectinib or a salt thereof and the inhibitor for gel-like substances is from 100 : 30 to 100 : 90 in terms of free form, and/or a weight ratio of the alectinib or a salt thereof and the surfactant and the inhibitor for gel-like substances is from 100 : 3 : 30 to 100 : 50 : 90 in terms of free form, and wherein the syrup has AUC and Cₘₐₓ equivalent to or greater than a capsule containing the same amount of alectinib or a salt thereof as the syrup;
a syrup or dry syrup containing alectinib or a salt thereof a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, acetic acid, maleic acid, and trisodium citrate; a preservative selected from sodium benzoate, paraoxybenzoic acid esters, chlorobutanol, benzyl alcohol, phenethyl alcohol, dehydroacetic acid, sorbic acid, and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant selected from the group consisting of carmellose calcium, crospovidone, sodium starch glycolate, and sodium croscarmellose; a binder selected from the group consisting of hydroxypropyl cellulose, hydroxypropyl methyl cellulose, methyl cellulose, povidone (polyvinylpyrrolidone), and gum arabic powder; a sweetener selected from the group consisting of sucrose, mannitol, fructose, lactose, sodium saccharin, aspartame, xylitol, erythritol, sucralose, and acesulfame potassium; and a flavor selected from the group consisting of a strawberry flavor, a raspberry flavor, an orange flavor, an apple flavor, a cherry black flavor, and a yogurt flavor, wherein the syrup has bioequivalence to a capsule containing the same amount of alectinib or a salt thereof as the syrup;
a syrup or dry syrup containing alectinib or a salt thereof; a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, and maleic acid; a preservative selected from sodium benzoate and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant that is crospovidone; a binder that is hydroxypropyl cellulose; a sweetener selected from the group consisting of sodium saccharin, sucralose, and acesulfame potassium; and a flavor selected from the group consisting of a strawberry flavor, a raspberry flavor, an orange flavor, and a cherry black flavor, wherein the syrup has bioequivalence to a capsule containing the same amount of alectinib or a salt thereof as the syrup;
a syrup or dry syrup containing alectinib or a salt thereof; a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, and maleic acid; a preservative selected from sodium benzoate and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant that is crospovidone; a binder that is hydroxypropyl cellulose; a sweetener selected from the group consisting of sodium saccharin, sucralose, and acesulfame potassium; and a flavor selected from the group consisting of a strawberry flavor, a raspberry flavor, an orange flavor, and a cherry black flavor, wherein a weight ratio of the alectinib or a salt thereof and the inhibitor for gel-like substances is from 100 : 30 to 100 : 90 in terms of free form, and/or a weight ratio of the alectinib or a salt thereof and the surfactant and the inhibitor for gel-like substances is from 100 : 3 : 30 to 100 : 50 : 90 in terms of free form, and wherein the syrup has bioequivalence to a capsule containing the same amount of alectinib or a salt thereof as the syrup;
a syrup or dry syrup containing alectinib or a salt thereof; a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, acetic acid, maleic acid, and trisodium citrate; a preservative selected from sodium benzoate, paraoxybenzoic acid esters, chlorobutanol, benzyl alcohol, phenethyl alcohol, dehydroacetic acid, sorbic acid, and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant selected from the group consisting of carmellose calcium, crospovidone, sodium starch glycolate, and sodium croscarmellose; a binder selected from the group consisting of hydroxypropyl cellulose, hydroxypropyl methyl cellulose, methyl cellulose, povidone (polyvinylpyrrolidone), and gum arabic powder; a sweetener selected from the group consisting of sucrose, mannitol, fructose, lactose, sodium saccharin, aspartame, xylitol, erythritol, sucralose, and acesulfame potassium; and a flavor selected from the group consisting of a strawberry flavor, a raspberry flavor, an orange flavor, an apple flavor, a cherry black flavor, and a yogurt flavor, wherein the syrup has an enhanced oral bioavailability compared to a capsule containing the same amount of alectinib or a salt thereof as the syrup;
a syrup or dry syrup containing alectinib or a salt thereof; a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, and maleic acid; a preservative selected from sodium benzoate and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant that is crospovidone; a binder that is hydroxypropyl cellulose; a sweetener selected from the group consisting of sodium saccharin, sucralose, and acesulfame potassium; and a flavor selected from the group consisting of a strawberry flavor, a raspberry flavor, an orange flavor, and a cherry black flavor, and wherein the syrup has 110 to 160% enhanced oral bioavailability compared to a capsule containing the same amount of alectinib or a salt thereof as the syrup;
a syrup or dry syrup containing alectinib or a salt thereof; a surfactant that is sodium lauryl sulfate; a gelling inhibitor selected from the group consisting of calcium chloride, sodium chloride, potassium chloride, sodium sulfate, calcium sulfate, calcium dihydrogen phosphate, sodium hydroxide, calcium hydroxide, and sodium bicarbonate; a pH adjusting agent selected from the group consisting of hydrochloric acid, citric acid, tartaric acid, and maleic acid; a preservative selected from sodium benzoate and potassium sorbate; a thickener selected from the group consisting of xanthan gum, hydroxyethyl cellulose, and sodium carboxymethyl cellulose; an excipient selected from the group consisting of starch, lactose hydrate, and crystalline cellulose; a disintegrant that is crospovidone; a binder that is hydroxypropyl cellulose; a sweetener selected from the group consisting of sodium saccharin, sucralose, and acesulfame potassium; and a flavor selected from the group consisting of a strawberry flavor, a raspberry flavor, an orange flavor, and a cherry black flavor, wherein a weight ratio of the alectinib or a salt thereof and the inhibitor for gel-like substances is from 100 : 30 to 100 : 90 in terms of free form, and/or a weight ratio of the alectinib or a salt thereof and the surfactant and the inhibitor for gel-like substances is from 100 : 3 : 30 to 100 : 50 : 90 in terms of free form, and wherein the syrup has 110 to 160% enhanced oral bioavailability compared to a capsule containing the same amount of alectinib or a salt thereof as the syrup.

The syrup of the present invention can be used as a therapeutic agent for childhood cancer.

Examples of the childhood cancer include pediatric malignant solid tumors and malignant lymphomas.

Examples of the pediatric malignant solid tumors include inflammatory myofibroblastic tumors, rhabdomyosarcoma, Ewing sarcoma family of tumors (Ewing sarcoma of the bone and other tissues, primitive neuroectodermal tumors (PNET), Askin's tumors (PNET occurring primarily in the chest wall), etc.), sarcomas such as leiomyosarcoma, central nervous system tumors such as neuroblastoma, glioma, neuroblastoma, and malignant peripheral nerve sheath tumors, cutaneous tumors such as malignant melanomas and Spitz tumors, retinoblastoma, lung cancers such as non-small cell lung cancer, renal cell carcinoma, anaplastic thyroid carcinoma, thymic carcinoma, and ovarian cancer, as well as epithelial tumor of the gallbladder and extrahepatic bile ducts, thyroid cancer, germ cell tumors, and malignant fibrous histiocytoma.

Malignant melanomas include (1) acral lentiginous melanoma (ALM), (2) superficial spreading melanoma (SSM), (3) nodular melanoma (NM), and (4) lentigo maligna melanoma (LMM).

Spitz tumors include malignant Spitz tumors, juvenile malignant melanoma, Spitzoid melanoma, and Spitz nevus-like melanoma.

Preferred examples of the childhood cancer include inflammatory myofibroblastic tumors, neuroblastoma, thymic carcinoma, ovarian cancer, anaplastic thyroid carcinoma, neuroblastoma, Spitz tumor, malignant melanoma, rhabdomyosarcoma, Ewing's sarcoma, retinoblastoma, and glioma of the central nervous system.

As the childhood cancer, inflammatory myofibroblastic tumors, diffuse large B-cell lymphoma, and neuroblastoma are preferred.

The syrup of the present invention can also be used to treat childhood cancers such as thymic carcinoma, ovarian cancer, Spitz tumor, malignant melanoma, retinoblastoma, diffuse large B-cell lymphoma, or rare histological subtypes of stomach cancer, bowel cancer, breast cancer, or liver cancer, which have ALK abnormality.

Preferably, the syrup of the present invention can be used to treat the above childhood cancers having a prior treatment failure, or with refractory or recurrent lesions.

"Prior treatment failure" means a case in which the prior treatment was ineffective and discontinued. Examples of the prior treatment include chemotherapy with other agents having similar or different mechanisms of action, radiation therapy, and surgery. Specific examples include cases in which resistance is developed by the administration of crizotinib. "Recurrent" means that the optimal response to the most recent treatment was a complete or partial remission. "Refractory" means that the optimal response to the most recent treatment was stable or advanced. Pathological conditions such as metastatic, curatively unresectable are also included.

Further another aspect of the present invention includes a pharmaceutical composition containing a poorly water-soluble agent, an anionic surfactant, and an inhibitor for gel-like substances. By allowing an inhibitor for gel-like substances to be present together in the composition, it is possible to obtain a composition of which the dissolution property of the poorly water-soluble agent or the palatability is improved compared to the composition that does not contain the inhibitor for gel-like substances. Preferably, the poorly water-soluble agent is a poorly water-soluble basic agent.

"Poorly water-soluble" refers to a state in which the solubility in the solvent, especially in water, a buffer solution, or a gastrointestinal fluid is 1 mg/ml or less, more preferably 100 µg/ml or less, still more preferably 10 µg/ml or less, especially preferably 1 µg/ml or less, most preferably 0.1 µg/ml or less. It is preferred that the agent is poorly water-soluble in any solvent having a pH of 7 or less, more preferably poorly water-soluble in any solvent having a pH of 4 to 7, and further preferably poorly water-soluble in a solvent having a pH of 4 and/or pH of 7. However, in the case of a compound having a basic group in a molecule, it is preferred that the agent is poorly water-soluble in any solvent having a pH of 7 or more, more preferably poorly water-soluble in any solvent having a pH of 7 to 9, and further preferably poorly water-soluble in a solvent having a pH of 7 and/or pH of 9. Solvents for measuring the solubility are not particularly limited. Examples of the solvent having pH 4 include an acetate buffer and a citrate buffer. Examples of the solvent having pH 5 include an acetate buffer, a citrate buffer, and a phosphate buffer. Examples of the solvent having pH 7 include water and a phosphate buffer. Examples of the solvent having pH 9 include a carbonate buffer. The measured temperature of the solubility is, in any case, preferably 20 to 40°C, more preferably 37°C.

A "basic agent" has at least one basic group, for example, a primary amino group (-NH2), a secondary amino group (imino group, -NH-), a tertiary amino group (>N-), an amide group, a basic nitrogen-containing heterocyclic group (a pyrrolyl group, an imidazolyl group, a pyrazolyl group, a pyrazinyl group, a purinyl group, a quinolyl group, a pyridyl group, a piperidine group, a piperidyl group, a piperazinyl group, a triazolo group, and the like). The amino group also includes a hydrazino group (-NH-NH2), a hydrazo group (-NH-NH-), and the like. The basic agent may have at least one basic group and may have multiple basic groups of the same or different types. The agent may also form a salt (e.g., a salt with an inorganic acid such as hydrochloric acid, sulfuric acid, or phosphoric acid; organic carboxylic acid such as acetic acid, tartaric acid, citric acid, fumaric acid, or maleic acid; or organic sulfonic acid such as mesylic acid). The cationic or basic agent also includes a drug whose metabolite or prodrug expressing activity in a living body being cationic or basic.

The poorly water-soluble agent may also form a salt (e.g., a salt with an inorganic acid such as hydrochloric acid, sulfuric acid, or phosphoric acid; organic carboxylic acid such as acetic acid, tartaric acid, citric acid, fumaric acid, or maleic acid; or organic sulfonic acid such as mesylic acid).

The use of the poorly water-soluble agent is not particularly limited, and may be, for example, a drug that acts on the central nervous system, autonomic nervous system, respiratory system, circulatory system, digestive system, metabolic system, or the like, or may be a hematologic or hematopoietic drug, an ophthalmic or otolaryngologic drug, an in vivo active substance (autacoid), or the like. Specific types thereof include an antipyretic agent, an analgesic agent, an anti-inflammatory drug, a hypnosis/sedation drug, an anti-rheumatic drug, an anti-depressant, an anti-epileptic agent, an anti-dizziness agent, an anti-allergic agent, a cardiotonic drug, a beta-blocker, a calcium antagonist, an anti-arrhythmic agent, a diuretic drug, an anti-angina drug, a heart failure therapeutic drug, a myocardial infarction therapeutic drug, an anti-hypertensive agent (hypertensive therapeutic drug), a peripheral circulatory disturbance therapeutic drug, a vasopressor (hypotensive therapeutic drug), a bronchodilator drug, an asthma therapeutic drug, an anti-tuberculosis drug, a diabetes therapeutic drug, a diabetic complication therapeutic drug, a hyperlipidemia therapeutic drug, a hyperuricemia therapeutic drug, a cough suppressant or expectorant, a peptic ulcer therapeutic agent, a thyroid disease therapeutic drug, a prostate hypertrophy therapeutic drug, an anti-cancer drug, an osteoporosis therapeutic drug, an Alzheimer's disease therapeutic drug, an antibiotic, vitamins, an anti-plasmin agent, and the like.

Specific examples of the poorly water-soluble drug include antipyretic, analgesic, and anti-inflammatory drugs (for example, an antipyretic analgesic drug such as dimethothiazine mesylate, a headache drug such as dihydroergotamine mesylate, lomeridine hydrochloride, sumatributane succinate, and an anti-inflammatory drug such as phenamic acid, mefenamic acid, fructafenin, proglumethacin maleate, epirizole, and thiaramide hydrochloride), an anti-rheumatic drug (such as penicillamine or methotrexate), a hyperuricemia therapeutic drug (such as allopurinol), a hypnosis/sedation drug (such as rilmazafone hydrochloride, zolpidem tartrate), an anti-depressant (such as nortriptyline hydrochloride, imipramine hydrochloride, amitriptyline hydrochloride, clomipramine hydrochloride, fluvoxamine maleate, milnacipran hydrochloride), an anti-dizziness agent (such as isoprenaline hydrochloride, betahistine mesylate), an anti-allergic agent (an anti-histamine agent such as diphenhydramine hydrochloride, diphenylpyraline theocurate, clemastine fumarate, chlorphenylamine maleate, alimemazine tartrate, and promethazine hydrochloride; and an histamine H1 antagonist (or basic anti-allergic agent) such as ketotifin fumarate, azelastine hydrochloride, and epinastine hydrochloride), a cardiotonic drug (such as denopamine, isoprenaline hydrochloride), an anti-angina drug (such as nicorandil, ethaphenone hydrochloride, dipyridamol, trapidyl, trimethadidine hydrochloride), a beta-blocker (such as propranolol hydrochloride, diphenidol hydrochloride, bufetolol hydrochloride, bupranolol hydrochloride, bopindolol malonate, oxprenolol hydrochloride, alprenolol hydrochloride, indenolol hydrochloride, acebutolol hydrochloride, celiprolol hydrochloride), a calcium antagonist (such as manidipine hydrochloride, benidipine hydrochloride, amlodipine besylate, verapamil hydrochloride, diltiazem hydrochloride), an anti-arrhythmic agent (such as aprindine hydrochloride, pilsicainide hydrochloride, propafenone hydrochloride, amiodarone hydrochloride, nifecalant hydrochloride, sotalol hydrochloride, bepridyl hydrochloride), a diuretic drug (such as hydrochlorothiazide, penflutizide, benzylhydrochlorothiazide, bumethanide, azosemide, triamterene), an anti-hypertensive agent (a sympatholytic such as clonidine hydrochloride, methyldopa, guanabens acetate, guanfacin hydrochloride, reserpine, prazosine hydrochloride, bunazosin hydrochloride, terazosin hydrochloride, or doxazosin mesylate, a vasodilator such as hydralazine hydrochloride, budralazine, todralazine hydrochloride, and cadralazine, an ACE inhibitor such as enalapril maleate, delapril hydrochloride, lisinopril, benazepril hydrochloride, and angiotensin I receptor antagonists such as candesartan cilexetil and valsartan), a peripheral circulatory disturbance therapeutic drug (such as inositol hexanicotinate, hepronicate, tolazoline hydrochloride, isoxprine hydrochloride), a vasopressor (such as metaraminol bitartrate, methoxamine hydrochloride, midodrine hydrochloride, amezinium methyl sulfate, ethyllephrine hydrochloride, phenylephrine hydrochloride), a bronchodilator and an asthma therapeutic drug (such as a β2-adrenergic receptor stimulators such as ephedrine hydrochloride, methylephedrine hydrochloride, isoprenaline hydrochloride, orciprenaline sulfate, chlorprenaline hydrochloride, salbmol hydrochloride, terbutaline hydrochloride, formoterol fumarate, tulobuterol hydrochloride, phenoterol hydrobromide, procaterol hydrochloride, and clenbuterol hydrochloride, xanthine derivatives such as theophylline, aminophylline, choline theophylline, and proxyphylline), a cough suppressant (such as dimemorfan phosphate, tipepidine hibenzate, oxerazine citrate, dextromethorphan hydrobromide, pentoxyverine citrate, cloperastine, or benproperine phosphate), a diabetes therapeutic drug (such as tolbutamide, acetohexamide, glybenchlamide, glymepyrid, buformin hydrochloride, ensanmetformin, pioglitazone hydrochloride, or boglibose), or an expectorant (such as L-methylcistine hydrochloride, ambroxol hydrochloride, or bromhexine hydrochloride), a peptic ulcer therapeutic agent (such as an H2 receptor antagonist such as cimetidine, ranitidine hydrochloride, famotidine, a proton pump inhibitor such as lansoprazole, omeprazole, a muscarinic receptor antagonist such as pyrenezepine hydrochloride), an antibiotic (such as clarithromycin, kitasamycin, josamycin, midecamycin, roxistamycin, azithromycin), a narcotic (such as amphetamine, meperidine), vitamins [vitamin B1 such as thiamine hydrochloride, thiamine nitrate, dicethiamine hydrochloride, cicothiamine, benfotiamine, bisibutiamine, furusultiamine, prosultiamine, octotiamine, bisbenthiamine, thiamine disulfide; vitamin B2 such as riboflavin, riboflavin sodium phosphate, riboflavin butyrate, flavin adenine dinucleotide sodium; vitamin B6 such as pyridoxine hydrochloride, pyridoxine acetate, and pyridoxal phosphate; nicotinic acid such as nicotinic acid, and nicotinamide; vitamin B12 such as mecobalamin, cyanocobalamin, hydroxocobalamin (such as hydroxocobalamin hydrochloride, and hydroxocobalamin acetate), and methylcobalamin; folic acid, pantothenic acid, biotin, vitamin P (such as hesperidin)], an anti-plasmin agent (such as epsilon-aminocaproic acid, tranexamic acid), an anti-cholinergic agent that is a poorly water-soluble basic drug having a morpholine backbone (mosapride citrate) and an morpholine-based anti-fungal drug (amorolfine hydrochloride salt).

These poorly water-soluble agents may be used alone or in combination of two or more, depending on the purpose of prevention or treatment, or the like.

The present invention also includes:
a composition consisting of a poorly water-soluble agent, an anionic surfactant, and an inhibitor for gel-like substances, wherein the composition has a better dissolution property of the agent compared to the composition that does not contain the inhibitor for gel-like substances;
a composition consisting of a poorly water-soluble agent, an anionic surfactant, and an inhibitor for gel-like substances, wherein the composition has a better medicine-palatability compared to the composition that does not contain the inhibitor for gel-like substances;
the composition, wherein the composition is a syrup; and
the composition, wherein the composition is a dry syrup.

The present invention further includes:
a method of improving the dissolution property of an agent by allowing an inhibitor for gel-like substances to be present together in the composition containing a poorly water-soluble agent and an anionic surfactant, compared to the composition that does not contain the inhibitor for gel-like substances;
a method of improving the fluidity and/or palatability of an preparation by allowing an inhibitor for gel-like substances to be present together in a composition containing a poorly water-soluble agent and an anionic surfactant, compared to the composition that does not contain the inhibitor for gel-like substances; and
a method of producing a preparation, including a step of allowing an inhibitor for gel-like substances to be present together in a composition containing a poorly water-soluble agent and an anionic surfactant, wherein the preparation has a better fluidity and/or palatability compared to the composition that does not contain the inhibitor for gel-like substances.

During the study in the present invention of a syrup containing alectinib or a salt thereof, which is a poorly water-soluble basic drug having a morpholine backbone, and an anionic surfactant, there is a problem that a gel is formed when the preparation is suspended due to an ionic interaction between alectinib or a salt thereof and an anionic surfactant, causing a decrease in fluidity and/or palatability. To deal with this problem, it has been confirmed that the formation of gel when the preparation is suspended can be suppressed by allowing an inhibitor for gel-like substances to be present together with alectinib or a salt thereof and the anionic surfactant.

A preparation having good dissolution property can be obtained by allowing an inhibitor for gel-like substances to be present together in a preparation containing alectinib or a salt thereof and a surfactant. "Good dissolution property" means that the following dissolution property is obtained: the dissolution rate of the preparation is 60% or more after 60 minutes when the dissolution test is performed by the paddle method of 50 revolutions per minute (rpm) using 900 mL of a solution of hydrochloric acid/sodium hydrochloride (pH 1.8) containing 4% polyoxyethylene lauryl ether as the test solution.

The above test solution is preferably a colorless and clear solution obtained by dissolving 20.0 g of sodium chloride in 10,000 mL of water, and adjusting the pH to about 1.8 with 37% hydrochloric acid to obtain a solution, and adding about 400.0 g of polyoxyethylene lauryl ether to the solution.

### [Examples]

Hereinafter, the present invention will be described in more detail based on Examples, but the present invention is not limited by these. In Examples 1 to 32, NIKKOL SLS (Nikko Chemicals Co., Ltd.) was used as sodium lauryl sulfate.

### Reference Example 1: Formation of gel-like substances

### (Production of Preparations)

A suspension was prepared according to the amounts of each component listed in Table 1. About 85% of the total amount of purified water was added to a glass beaker, and sodium lauryl sulfate and alectinib hydrochloride (hereinafter, Compound A) were added while stirring with a magnetic stirrer, and the mixture was stirred until completely dispersed. The pH of the suspension was measured, and a citric acid solution was further added to adjust the pH to 4.0, and then the total amount of the suspension was filled up to 60 mL with the purified water.

**Table 1. Formulation amount (g) per preparation**

| **Component** | **Reference Example 1** |
|---|---|
| Compound A | 3.8718 |
| Sodium lauryl sulfate | 1.8000 |
| Citric acid*¹ | 0.0704 |
| **Purified water** | 60 mL |

| | |
|---|---|
| *¹ Trade name: Citric Acid, Supplier: Merck KGaA | |

### (Preparation Evaluation and Results)

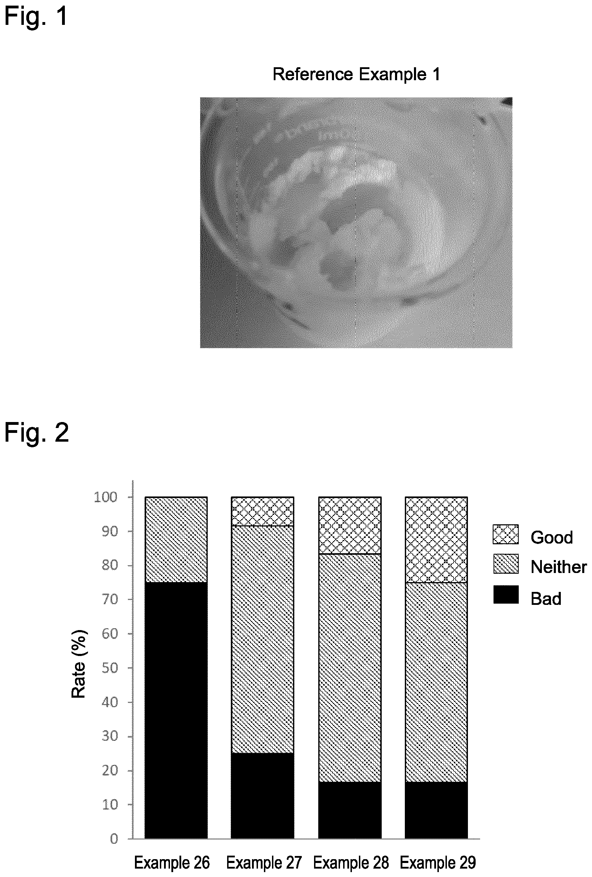

For Reference Example 1, the appearance of the suspension at immediately after preparing the suspension and after 2 days at room temperature was visually observed. Figure 1 shows a photograph of the appearance after 2 days at room temperature.

The suspension of Reference Example 1 increased the fluidity after 5 minutes of the preparation, and gel-like substances were formed. As shown in Figure 1, the suspension maintained a high viscosity state even after 2 days at room temperature, and it was confirmed that the fluidity of the suspension was significantly decreased. The formation of a gel-like substance refers to a significant decrease in fluidity due to Compound A and sodium lauryl sulfate in the suspension.

### Test Example 1: Suppression effect on gel-like substance formation by salt

### (Production of Preparations)

Suspensions were prepared according to the amounts of each component listed in Table 2. Respective substances used for the saturated aqueous solution of X contained in the formulation were listed in Table 3. Sodium lauryl sulfate was added to a glass bottle, then 20 mL of each saturated solution was added. Compound A was then added and mixed until fully dispersed to prepare a suspension.

**Table 2. Formulation amount (mg) per preparation**

| **Component** | **Comparative Example 1, Examples 1 to 10** |
|---|---|
| Compound A | 323 |
| Sodium lauryl sulfate | 75.0 |
| **Saturated aqueous solution of X*** | 20 mL |

| | |
|---|---|
| ' Additive listed in Table 3. | |

**Table 3. List of substances used for X**

| Comparative Example/Example | Substance name | Trade name | Supplier |
|---|---|---|---|
| Comparative Example 1 | Purified water | - | - |
| Example 1 | Calcium chloride | Calcium chloride dihydrate | Merck KGaA |
| Example 2 | Sodium chloride | Sodium chloride | FUJIFILM Wako Pure Chemical Corporation |
| Example 3 | Potassium chloride | Potassium chloride | Merck KgaA |
| Example 4 | Sodium sulfate | Sodium sulfate | FUJIFILM Wako Pure Chemical Corporation |
| Example 5 | Sodium hydroxide | Sodium hydroxide (UE-E) | Kanto Chemical Co., Inc. |
| Example 6 | Sodium bicarbonate | Sodium bicarbonate (only for production) | FUJIFILM Wako Pure Chemical Corporation |
| Example 7 | Calcium sulfate | Calcium sulfate | FUJIFILM Wako Pure |
| | | | Chemical Corporation |
| Example 8 | Calcium hydroxide | Calcium hydroxide | FUJIFILM Wako Pure Chemical Corporation |
| Example 9 | Calcium dihydrogen phosphate | Calcium dihydrogen phosphate | FUJIFILM Wako Pure Chemical Corporation |
| Example 10 | Sodium dihydrogen phosphate | Sodium dihydrogen phosphate, dodecahydrate | FUJIFILM Wako Pure Chemical Corporation |

**Table 4. Suppression effect of each substance on gel-like substance formation**

| Comparative Example/Exam ple | Substance name | Results |
|---|---|---|
| Comparative Example 1 | Purified water | - |
| Example 1 | Calcium chloride | + |
| Example 2 | Sodium chloride | + |
| Example 3 | Potassium chloride | + |
| Example 4 | Sodium sulfate | + |
| Example 5 | Sodium hydroxide | + |
| Example 6 | Sodium bicarbonate | + |
| Example 7 | Calcium sulfate | + |
| Example 8 | Calcium hydroxide | + |
| Example 9 | Calcium dihydrogen phosphate | + |
| Example 10 | Sodium dihydrogen phosphate | - |

### (Preparation Evaluation and Results)

For Comparative Example 1 and Examples 1 to 10, the appearance of the suspension after 1 day at room temperature was visually observed. The suppression effects of Examples 1 to 10 on gel-like substance formation are shown in Table 4. The following symbols used in Tables herein indicate the following meanings.
-: Gel-like substance formation is observed, or no suppression effect on gel-like substance formation is observed.
+: Suppression effect on gel-like substance formation is observed.

As shown in Table 4, in Examples 1 to 9, no gel formation was observed in the suspension prepared using the saturated aqueous solution of salts as X, and it was confirmed that the salts of Examples 1 to 9 exhibited a suppression effect on gel-like substance formation.

### Test Example 2: Compounding change

### (Production of Preparations)

Tablets were prepared according to the amounts of each component of the tablet formulations listed in Tables 5 and 6. For Y and Z contained in the formulation, each substance shown in Tables 7 and 8 was used. To a glass bottle, Compound A and sodium lauryl sulfate and each additive of Y at a ratio of 1 : 1 : 1, or Compound A and sodium lauryl sulfate and each additive of Z at a ratio of 10 : 10 : 1 were added. The mixture was mixed at 32 rpm for 5 minutes using a mixing machine, and then the mixture was molded with a 12 mm diameter round tablet mold using a static pressure tableting machine to obtain a tablet having a weight of 600 mg. A tablet of 600 mg of Compound A was similarly prepared as Comparative Example 2. Tablets were placed in a glass bottle and stored at 70°C/75%RH for 14 days to perform a storage test. For each stored sample, the content and the production amount of related substances were evaluated.

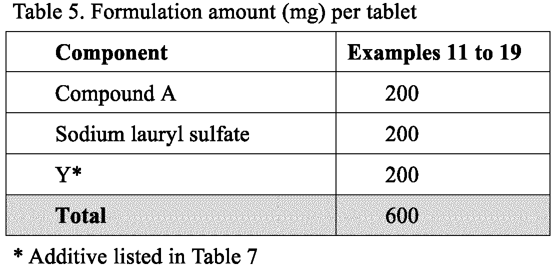

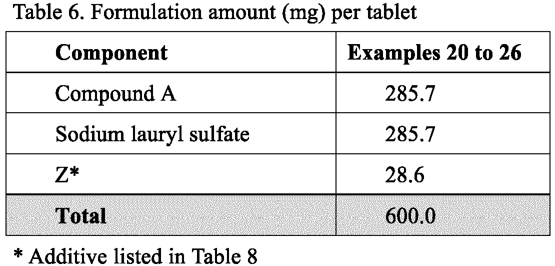

**Table 7. List of substances used for Y**

| Example | Substance name | Type | Trade name | Supplier |
|---|---|---|---|---|
| Example 11 | Citric acid | pH adjusting agent | Citric acid | Merck KGaA |
| Example 12 | Tartaric acid | pH adjusting agent | Tartaric acid | Merck KGaA |
| Example 13 | Maleic acid | pH adjusting agent | Maleic acid | Merck KGaA |
| Example 14 | Xanthan gum | Thickener | Xantural 75 | CP Kelco |
| Example 15 | Hydroxyethyl cellulose | Thickener | Natrosol 250HX | Ashland |
| Example 16 | Sodium carboxymethyl cellulose | Thickener | Blanose CMC 7H3SXF | Ashland |
| Example 17 | Sodium saccharin | Sweetener | Sodium saccharin | Merck KGaA |
| Example 18 | Sucralose | Sweetener | Sucralose | Merck KGaA |
| Example 19 | Acesulfame potassium | Sweetener | Acesulfame potassium | - |

**Table 8. List of substances used for Z**

| Example | Substance name | Type | Trade name | Supplier |
|---|---|---|---|---|
| Example 20 | Sodium benzoate | Preservative | Sodium benzoate | Merck KGaA |
| Example 21 | Potassium sorbate | Preservative | Potassium sorbate | Merck KGaA |
| Example 22 | Cherry black flavor | Flavor | Cherry Black Flavour 501027 A7P0504 | Firmenich |
| Example 23 | Raspberry flavor | Flavor | Raspberry Flavour 501199 AP0551 | Firmenich |
| Example 24 | Strawberry flavor | Flavor | Strawberry Flavouring Powder SC11929 | IFF |
| Example 25 | Orange flavor | Flavor | Orange Flavour SC611927 | IFF |

**Table 9. Compounding changes of Comparative Examples 2 and 3 and Examples 11 to 25**

| | | 70°C/75%RH for 14 days | |
|---|---|---|---|
| Comparative Example/Example | Substance name | Quantitative value (%) | Total amount of related substancess (%) |
| Comparative Example 2 | Compound A | 107.9 | 0.08 |
| Example 11 | Citric acid | 94.8 | 6.18 |
| Example 12 | Tartaric acid | 120.5 | 2.94 |
| Example 13 | Maleic acid | 83.8 | 9.42 |
| Example 14 | Xanthan gum | 96.4 | 0.08 |
| Example 15 | Hydroxyethyl cellulose | 102.4 | 0.09 |
| Example 16 | Sodium carboxymethyl cellulose | 106.8 | 0.09 |
| Example 17 | Sodium saccharin | 102.3 | 2.15 |
| Example 18 | Sucralose | 97.1 | 1.14 |
| Example 19 | Acesulfame potassium | 102.8 | 0.26 |
| Example 20 | Sodium benzoate | 102.8 | 0.07 |
| Example 21 | Potassium sorbate | 99.2 | 0.72 |
| Example 22 | Cherry black flavor | 104.2 | 0.38 |
| Example 23 | Raspberry flavor | 101.8 | 0.47 |
| Example 24 | Strawberry flavor | 107.0 | 0.09 |
| Example 25 | Orange flavor | 100.6 | 0.11 |

### (Preparation Evaluation and Results)

In Examples 11 to 25, additives with fewer compounding changes were selected from the viewpoint of the content and the total amount of related substances. For the pH adjusting agent, tartaric acid, which had the smallest increase in the total amount of related substances compared to citric acid and maleic acid, was selected as an additive to be compounded in the preparation. For a thickener and a flavor, there was no significant change in the content and the total amount of related substancess in any additive, and it was confirmed that all were acceptable as an additive to be compounded in the preparation. For a sweetener, sucralose and acesulfame potassium were confirmed to have a lower total amount of related substances compared to saccharin sodium. For a preservative, sodium benzoate, which had a smaller increase in the total amount of related substances compared to potassium sorbate, was selected as an additive to be compounded in the preparation.

### Test Example 3: Taste test in human

### (Production of Preparations)

A solvent was prepared according to the amounts of each component listed in Table 10. 200 mL of purified water was added to a glass beaker, and sodium benzoate, sodium lauryl sulfate, sucralose, and strawberry flavor were added in this order while stirring with a magnetic stirrer, and the mixture was stirred until completely dissolved. Then, calcium chloride dihydrate was added, and the mixture was stirred for about 10 minutes. The pH of this solvent was measured, and 0.1 M hydrochloric acid was further added to adjust the pH to 2.50 to 4.0, and then the total amount of the solvent was filled up to 300 mL with the purified water. Compound A was added to 5 mL of the prepared solvent to prepare a suspension.

**Table 10. Formulation amount (mg) per preparation**

| | Example 26 | Example 27 | Example 28 | Example 29 |
|---|---|---|---|---|
| Compound A | 332.65 | 332.65 | 332.65 | 332.65 |
| Sodium benzoate | 11.50 | 11.50 | 11.50 | 11.50 |
| Sodium lauryl sulfate | 75.00 | 75.00 | 75.00 | 150.00 |
| Sucralose | - | 15.00 | 15.00 | 15.00 |
| Strawberry flavor | - | - | 12.50 | 12.50 |
| Calcium chloride dihydrate | 250.00 | 250.00 | 250.00 | 250.00 |
| 0.1 M Hydrochloric acid | Appropriate amount | Appropriate amount | Appropriate amount | Appropriate amount |
| Purified water | 5 mL | 5 mL | 5 mL | 5 mL |

For Examples 26 to 29, 12 subjects took each suspension in their mouths, and performed overall evaluation by scoring the taste evaluation with nine levels. The scores were summarized into 3 levels of 1 to 3 as "Dislike ", 4 to 6 as "Neutral ", and 7 to 9 as "Like", and the results are shown in Figure 2.

### (Preparation Evaluation and Results)

As shown in Figure 2, Examples 28 and 29 were confirmed to have improved palatability of suspension and acceptable levels of taste by adding a compounding-stable sweetener and flavor.

### Test Example 4: Dry syrup

### (Production of Preparations)

According to the amount of each component listed in Table 11, the granule formulation components were weighed, and mixed and granulated using a highspeed stirring granulator with addition of purified water. The obtained granulated powder was subjected to a particle size selector with a screen of 3.962 mm in size diameter to yield granules. The granules were dried in a dryer at a temperature of 50°C until the drying weight loss was 3% or less. The obtained dried powder was subjected to a particle size selector with a screen of 1.143 mm in size diameter to yield granules. The granules were further mixed with an extra-granules to obtain a dry syrup. Suspensions were prepared by reconstituting 2,000 mg of the dry syrup with 10 mL of purified water, or 3,000 mg of the dry syrup with 127.5 mL of purified water, and the appearance and the dissolution property were examined.

**Table 11. Formulation amount (mg) of 2000 mg of dry syrup**

| | Formulation component | Example 30 | Example 31 |
|---|---|---|---|
| **Intra-granular component** | | | |
| | Compound A | 645.3 | 645.3 |
| | Sodium benzoate | 5.0 | 5.0 |
| | Sodium lauryl sulfate | 300.0 | 150.0 |
| | Sucralose | 30.0 | 30.0 |
| | Tartaric acid | 12.5 | 12.5 |
| | Lactose hydrate | 164.7 | 314.7 |
| | Hydroxypropyl cellulose | 32.5 | 32.5 |
| | Crospovidone | 260.0 | 260.0 |
| | Calcium chloride dihydrate | 500.0 | 500.0 |
| | [Purified water]* | Appropriate amount | Appropriate amount |

| **Extra-glanular component** | | | |
|---|---|---|---|
| | Strawberry flavor | 25.0 | 25.0 |
| | Xanthan gum | 25.0 | 25.0 |
| | Total | 2000 | 2000 |

| | | | |
|---|---|---|---|
| * Purified water is removed by drying. | | | |

### (Preparation Evaluation and Results)

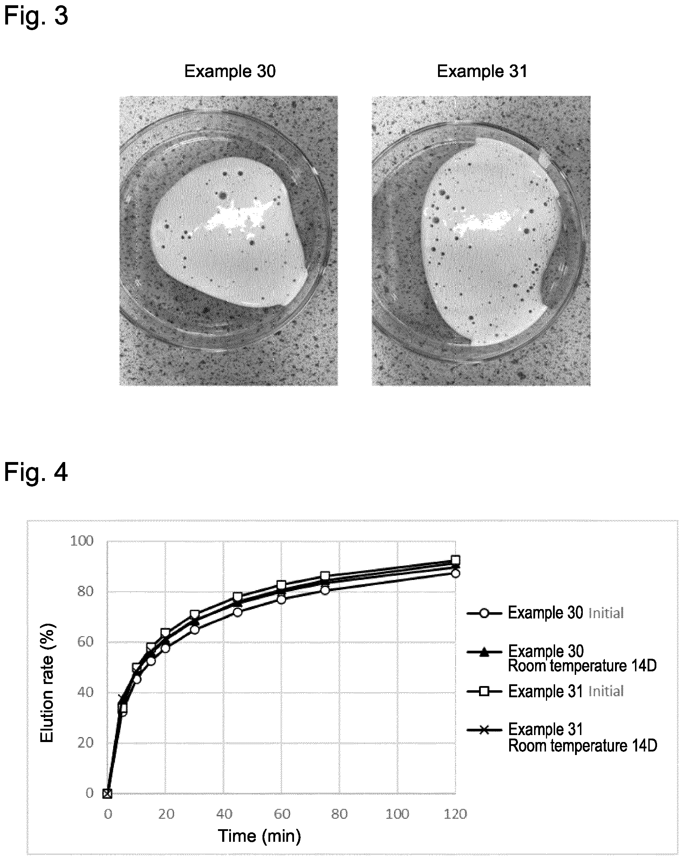

For Examples 30 and 31, the appearance of the suspension was visually observed. As shown in Figure 3, it was shown that allowing calcium chloride, which is a salt, to be present together can suppress gel-like substances and ensure the fluidity and/or palatability of the suspension.

### (Preparation Evaluation and Results)

For Examples 30 and 31, the dissolution profile when the test was performed by the paddle method at 50 revolutions per minute at 37°C with 900 mL of a solution of 4% polyoxyethylene lauryl ether (trade name: Brij(TM)35) hydrochloric acid/sodium chloride (pH 1.8) in the test solution is shown in Figure 4. The test solution is a colorless and clear solution obtained by dissolving 20.0 g of sodium chloride in 10,000 mL of water, and adjusting the pH to about 1.8 with 37% hydrochloric acid to obtain a solution, and adding about 400.0 g of polyoxyethylene lauryl ether to the solution.

As shown in Figure 4, by allowing calcium chloride, which is a salt, to be present together, no concerned reduction in elution was observed, and the dissolution property of 60% or more after 60 minutes was obtained in the elution test. It was thus confirmed good dissolution property of the suspension.

### Test Example 5: Relative bioavailability study of capsule and dry syrup

This is a single centre, open-label, non-randomised, single dose phase I clinical trial in healthy subjects, designed to investigate the PK, relative bioavailability of the capsule and the dry syrup.

For suspension administration of the dry syrup of the present invention, a suspension was prepared with 10 mL of water, and the bottle after administration was rinsed with 40 mL of water three times, and then the preparation was taken with a total of 240 mL of water, including the rinse water. Powder administration was performed without suspending, and the bottle after administration was rinsed 3 to 6 times with 40 mL of water, and then the preparation was taken with a total of 240 mL of water, including the rinse water. The capsule was taken by 4 capsules of ALECENSA(R) 150 mg (CHUGAI PHARMACEUTICAL CO., LTD.) with 240 mL of water.

A comparison of the dry syrup with an approved capsule was performed to verify PK and relative bioavailability and safety of the dry syrup. The relative bioavailability study specified formulations for the dry syrup having a serum concentration equivalent to or greater than the approved capsule.

In this study, the safety and tolerability of the dry syrups in healthy subjects were also evaluated based on the following evaluation items.
- Incidence, character, and severity of adverse events
- Standard 12-lead electrocardiogram (ECG) parameters
- Vital signs
- Clinical laboratory results (clinical chemistry test, blood test, urine test)
- Physical examination

In this study, two dry syrup of Examples 30 and 31 were evaluated with the adult daily dose of the approved 150 mg capsule as listed in Table 12 below as the control.

**Table 12. Formulation of Capsule**

| Component | Amounts (mg) |
|---|---|
| | Capsule (control) |
| Compound A (in terms of free form) | 645.30 (600.00) |
| Lactose monohydrate | 134.00 |
| Hydroxypropyl cellulose | 60.00 |
| Sodium lauryl sulfate | 300.00 |
| Carmellose calcium | 174.00 |
| Magnesium stearate | 6.70 |
| Total | 1320.00 |

The relative bioavailability after a single administration of the capsule and two types of the dry syrups was confirmed. A rest period of 10 days or more was provided between each administration. The effect of diet on exposure amounts was evaluated.

The preparations evaluated in each period are described in Table 13.

**Table 13. Preparation**

| Preparation | Dose | Administration | Number of subjects |
|---|---|---|---|
| Capsule (control) | 600mg | Under fasting | 26 |
| Suspension of dry syrup (Example 30) | 600mg | Under fasting | 13 |
| Suspension of dry syrup (Example 31) | 600mg | Under fasting | 13 |
| Powder of dry syrup (Example 31) | 600mg | Under fasting | 6 |

Venous blood samples were collected by indwelling cannula or venipuncture according to the time schedule shown in Table 14 below.

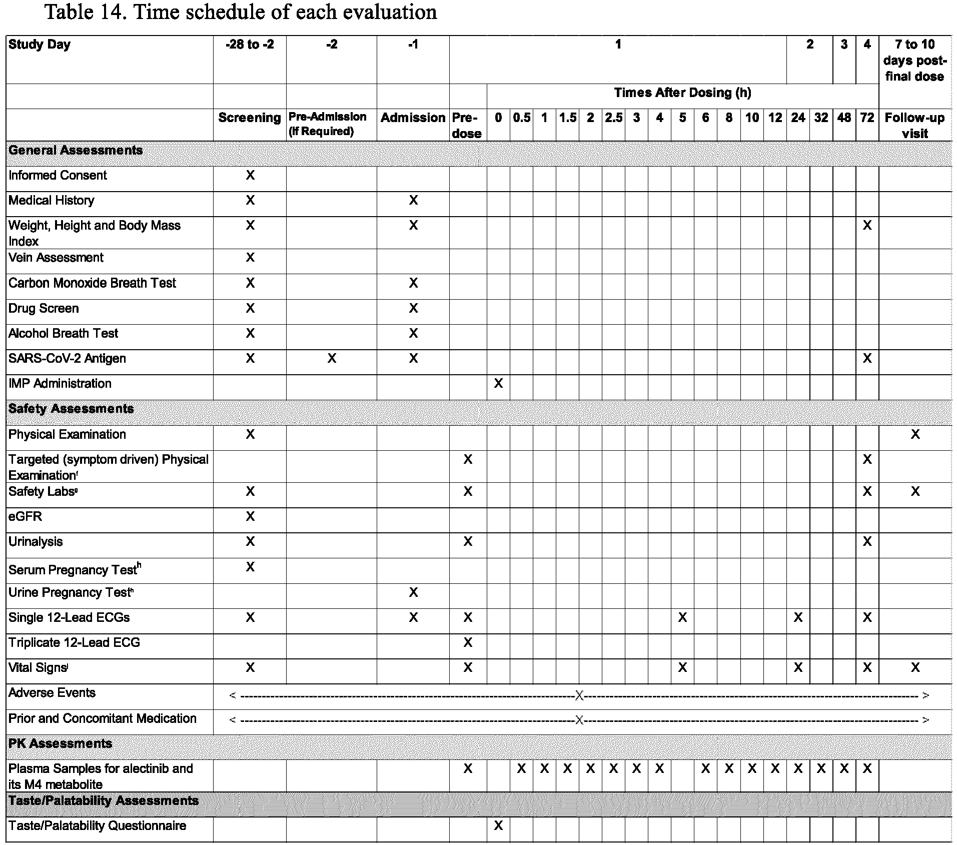

The plasma concentration of alectinib and its M4 metabolite in a venous blood sample was measured by LOMS/MS method in Q2 Solutions Biosciences LLC, and analyzed according to the method (Method 2) described in Bioanalysis (2016) 8(14), 1465-79.

The ratio of each dry syrup was shown in Table 15, with the geometric mean values of Cmax (ng/mL), AUC₍₀₋ₗₐₛₜ₎ (ng.h/mL), and AUC_{(0-inf)} (ng.h/mL) of the capsule taken as 100. The following symbols used in Tables herein indicate the following meanings.
↑ : The ratio of geometric mean value is 125% or more
→ : The ratio of geometric mean value is in the range of 80 to 125%

**Table 15. Ratio of geometric mean value of pharmacokinetic parameters when alectinib is orally administered in a single dose.**

| **Preparation** | **Cmax ratio (%)** | **AUC(0-last) ratio (%)** | **AUC(0-inf) ratio (%)** |
|---|---|---|---|
| Suspension of dry syrup (Example 30) | ↑ | ↑ | ↑ |
| Suspension of dry syrup (Example 31) | → | ↑ | ↑ |
| Powder of dry syrup (Example 31) | → | → | → |

When the suspensions of the dry syrups (Examples 30 and 31) were orally administered in a single dose under fasted conditon, the AUC and Cmax of Example 30 were increased compared to those of the capsule, and the Cmax of Example 31 was approximately equivalent and the AUC thereof was increased compared to those of the capsule.

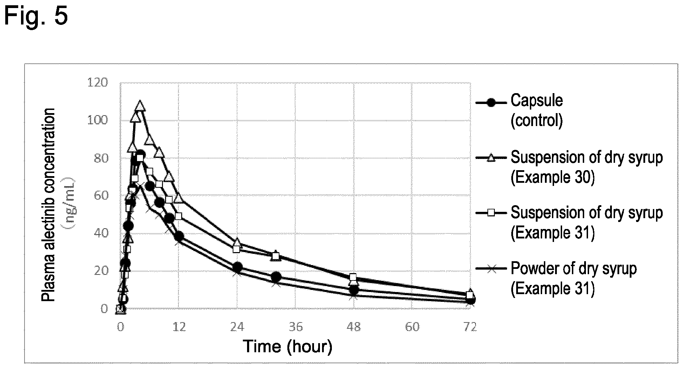

Figure 5 shows the changes in plasma concentration when capsule and dry syrup were administered in a single dose. A single administration of the suspension of the dry syrup (Examples 30 and 31) exhibited an equivalent or higher exposure compared to the capsule.

Furthermore, the suspension administration of the dry syrup (Example 31) exhibited an equivalent or higher exposure compared to the powder administration of the dry syrup (Example 31).

## Claims

1. A syrup containing alectinib or a salt thereof, wherein the syrup has a plasma concentration profile equivalent to or greater than a capsule containing the same amount of alectinib or a salt thereof as the syrup.

2. A syrup containing alectinib or a salt thereof, wherein the syrup has AUC and Cₘₐₓ equivalent to or greater than a capsule containing the same amount of alectinib or a salt thereof as the syrup.

3. A syrup containing alectinib or a salt thereof, wherein the syrup has bioequivalence to a capsule containing the same amount of alectinib or a salt thereof as the syrup.

4. The syrup according to any one of claims 1 to 3, further containing an inhibitor for gel-like substances.

5. The syrup according to claim 4, wherein a weight ratio of the alectinib or a salt thereof and the inhibitor for gel-like substances is from 100 : 30 to 100 : 90 in terms of free form.

6. The syrup according to any one of claims 1 to 3, further containing a surfactant and an inhibitor for gel-like substances.

7. The syrup according to claim 6, wherein a weight ratio of the alectinib or a salt thereof and the surfactant and the inhibitor for gel-like substances is from 100 : 3 : 30 to 100 : 50 : 90 in terms of free form.

8. A syrup containing alectinib or a salt thereof, a surfactant, and an inhibitor for gel-like substances.

9. The syrup according to claim 8, wherein the inhibitor for gel-like substances is a salt.

10. The syrup according to claim 9, wherein the salt is a water-soluble salt.

11. The syrup according to claim 9 or 10, wherein the salt is an inorganic salt or an organic salt.

12. The syrup according to claim 11, wherein the inorganic salt is selected from the group consisting of a chloride salt, a sulfate, a hydroxide salt, a carbonate, and a bicarbonate.

13. The syrup according to claim 11, wherein the organic salt is selected from the group consisting of a tartrate, an acetate, a citrate, an amino acid salt, an alginate, and a sorbate.

14. The syrup according to claim 10, wherein the salt is selected from the group consisting of calcium chloride, sodium chloride, potassium chloride, sodium sulfate, calcium sulfate, calcium dihydrogen phosphate, sodium hydroxide, calcium hydroxide, and sodium bicarbonate.

15. The syrup according to claim 8, wherein the surfactant is an anionic surfactant.

16. The syrup according to claim 15, wherein the anionic surfactant is sodium lauryl sulfate.

17. The syrup according to claim 8, further containing a pH adjusting agent.

18. The syrup according to claim 17, wherein the pH adjusting agent is tartaric acid.

19. The syrup according to claim 8, further containing one or more substances selected from a disintegrant, an excipient, and a binder.

20. The syrup according to claim 8, wherein a weight ratio of the alectinib or a salt thereof and the inhibitor for gel-like substances is from 100 : 30 to 100 : 90 in terms of free form.

21. The syrup according to claim 8, wherein a weight ratio of the alectinib or a salt thereof and the surfactant and the inhibitor for gel-like substances is from 100 : 3 : 30 to 100 : 50 : 90 in terms of free form.

22. The syrup according to claim 8, further containing one or more substances selected from a preservative, a thickener, a sweetener, and a flavor.

23. The syrup according to claim 22, wherein the thickener is selected from the group consisting of xanthan gum, hydroxyethyl cellulose, and sodium carboxymethyl cellulose; the sweetener is selected from the group consisting of sucrose, mannitol, fructose, lactose, sodium saccharin, aspartame, xylitol, erythritol, sucralose, and acesulfame potassium; and the flavor is selected from the group consisting of a strawberry flavor, a raspberry flavor, an orange flavor, an apple flavor, a cherry black flavor, and a yogurt flavor.

24. The syrup according to any one of claims 1 to 3 and 8, wherein the syrup is a dry syrup.
